# EUROPEAN PATENT APPLICATION

(11) **EP 2 468 280 A1**
(43) Date of publication of application: **27.06.2012**
(21) Application number: 10306480.4
(22) Date of filing: 21.12.2010
(51) Int. Cl.: A61K 31/443, A61K 38/17, A61P 9/10, A61P 13/12

(54) **Use of prokineticin 1 receptor agonists to promote the differentiation of epicardin+ progenitor cells**

(71) Applicant: Université de Strasbourg, 67000 Strasbourg (FR); Centre National de la Recherche Scientifique, 75016 Paris (FR)
(72) Inventor: The designation of the inventor has not yet been filed
(74) Representative: Gallois, Valérie

(57) **Abstract**

The present invention discloses a prokineticin receptor-1 agonist for use for promoting the differentiation of cardial epicardin+ progenitor cells into cardiomyocytes in a subject affected with a cardiac disease and/or the differentiation of renal epicardin+ progenitor cells into vasculogenic and/or glomerular cells in a subject affected with a renal disease.

SEQUENCE LISTING

<110>  Université de Strasbourg
       CNRS

<120>  Use of prokineticin 1 receptor agonists to promote the
       differentiation of epicardin+ progenitor cells

<130>  B1112EP

<160>  6

<170>  PatentIn version 3.3

<210>  1
<211>  21
<212>  DNA
<213>  Artificial

<220>
<223>  Mouse 36B4 forward primer

<400>  1
agattcggga tatgctgttg g                                                21


<210>  2
<211>  21
<212>  DNA
<213>  Artificial

<220>
<223>  Mouse 36B4 reverse primer

<400>  2
aaagcctgga agaaggaggt c                                                21


<210>  3
<211>  21
<212>  DNA
<213>  Artificial

<220>
<223>  Mouse PPAR alpha forward primer

<400>  3
acgatgctgt cctccttgat g                                                21


<210>  4
<211>  21
<212>  DNA
<213>  Artificial

<220>
<223>  Mouse PPAR alpha reverse primer

<400>  4
gtgtgataaa gccattgccg t                                                21


<210>  5
<211>  18
<212>  DNA
<213>  Artificial

<220>
<223>  Mouse PPAR gamma forward primer

<400>  5
ccattctggc ccaccaac                                                    18


<210>  6
<211>  21
<212>  DNA
<213>  Artificial

<220>
<223>  Mouse PPAR gamma reverse primer

<400>  6
aatgcgagtg gtcttccatc a                                                21

## Description

### Field of the Invention

The present invention relates to the fields of cardiology and nephrology. In particular, the present invention relates to the use of prokineticin receptor-1 agonists to treat a patient suffering from a cardiovascular disease, such as acute myocardial infarction, and/or suffering from a renal failure, in particular resulting from an ischemic renal disorder.

### Background of the Invention

Cardiovascular diseases are a major health risk throughout the industrialized world. Among these diseases, myocardial infarction, commonly known as a heart attack, is one of the most well-known types and is the most common cause of ischemia in heart. Myocardial infarction is caused by a sudden and sustained lack of blood flow to an area of the heart, commonly caused by narrowing of a coronary artery. Without adequate blood supply, the tissue becomes ischemic, leading to the death of myocytes and vascular structures. This area of necrotic tissue is referred to as the infarct site, and will eventually become scar tissue. Survival is dependent on the size of this infarct site, with the probability of recovery decreasing with increasing infarct size.

Current treatments for myocardial infarction focus on reperfusion therapy, which attempts to start the flow of blood to the affected area to prevent the further loss of tissue. These treatments may succeed in reestablishing the blood supply, however tissue damages that occurred before the reperfusion treatment began has been thought to be irreversible and organ transplantation may be needed to replace nonfunctional tissue.

Significant effort has been invested in stem-cell-based therapies and multipotent progenitor cells have been recently identified in the heart. Among these cells, epicardially derived cells (EPDC) are characterized as the stem cell-derived multipotential vascular progenitors (Wessels et al., 2004). EPDCs are positive for the epicardial-specific transcription factor, epicardin. They can either form endothelial cells, in response to a combination of myocardial vascular endothelial growth factor (VEGF) and basic fibroblast growth factor (bFGF) signaling, or differentiate into smooth muscle cells, on exposure to platelet-derived growth factor (PDGF), transforming growth factor β (TGF- β), and bone morphogenetic protein-2 (BMP-2) (Kruithof et al., 2006). Thymosin β4 and prokineticin-2 were identified as signaling factors for adult EPDC mobilization and differentiation into endothelial and smooth muscle cells promoting neovasculogenesis (Smart et al., 2007; Urayama et al., 2008).

However, there is still a strong need for improvement of stem-cell-based therapies for heart diseases, and in particular for a method for promoting cardiomyocyte regeneration.

### Summary of the Invention

The object of the present invention is to provide new therapeutic approaches for treating cardiac diseases and/or renal diseases. In particular, the present invention offers the possibility to treat patients with cardiac and/or renal necrosis, in particular due to ischemic diseases, and to provide a regeneration of necrotic areas.

In a first aspect, the present invention concerns a prokineticin receptor-1 agonist for use for promoting the differentiation of cardial epicardin+ progenitor cells (cEPPC) into cardiomyocytes in a subject affected with a cardiac disease and/or the differentiation of renal epicardin+ progenitor cells (rEPPC) into vasculogenic and/or glomerular cells in a subject affected with a renal disease. In an embodiment, the cardiac disease is selected from the group consisting of heart failure, myocardial infarction, ischemic heart disease, diabetes-mediated cardiovascular complications and the cardiorenal syndrome. In another embodiment, the renal disease is selected from the group consisting of chronic renal disease, renal artery stenosis, renal failure, acute kidney injury, acute-on-chronic renal failure, ischemic nephropathy, Churg-Strauss syndrome, Wegener's granulomatosis, diabetes-mediated renal complications and the cardiorenal syndrome.

In a second aspect, the present invention concerns a prokineticin receptor-1 agonist for use for preventing fat tissue development in heart and/or kidney in a subject, in particular in a subject affected with obesity or being susceptible to develop obesity.

In another aspect, the present invention concerns an *in vitro* or *ex vivo* method of producing cardiomyocytes, wherein said method comprises the step of contacting cardial epicardin+ progenitor cells with a prokineticin receptor-1 agonist. The present invention also concerns an *in vitro* or *ex vivo* method of producing glomerular cells, wherein said method comprises the step of contacting renal epicardin+ progenitor cells with a prokineticin receptor-1 agonist.

In an embodiment, the PKR1 agonist is prokineticin -2 or an active fragment thereof.

In another embodiment, the PKR1 agonist is a compound of formula (I) 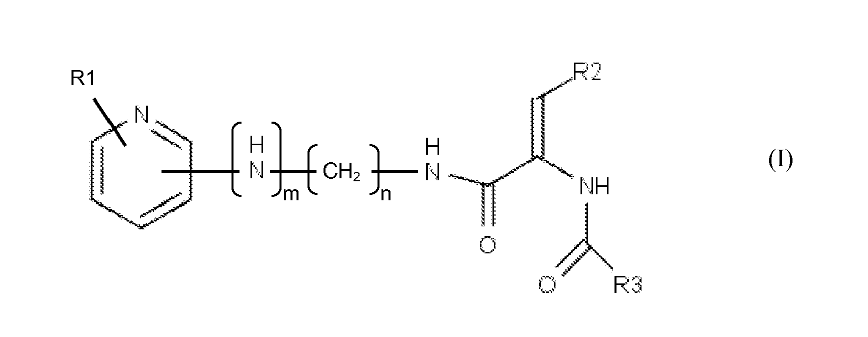 wherein
m is selected from 0 and 1;
n is selected from 0, 1 and 2;
R¹ is selected from the group consisting of a hydrogen atom; a halogen atom; a (C₁-C₆)-alkyl, a (C₂-C₆)-alkenyl and a (C₂-C₆)-alkynyl group, optionally substituted by at least one substituent selected from a hydroxyl, a carboxylic group, an aldehyde, a halogen atom and a thiol; a (C₆-C₁₂)-aryl; a 5 to 7-membered-ring heterocycle; a (C₁-C₆)-alkoxy, a (C₂-C₆)-acyl, a (C₂-C₆)-ester, a (C₁-C₆)-amine, a (C₁-C₆)-amide, a (C₁-C₆)-imine, a (C₁-C₆)-nitrile, a (C₁-C₆)-thioalkyl, a (C₁-C₆)-sulfone and a (C₁-C₆)-sulfoxide group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;
R² is selected from the group consisting of a (C₆-C₁₂)-aryl group and a 5 to 7-membered-ring heterocycle, preferably from phenyl and furan, optionally substituted by at least one substituent selected from the group consisting of a (C₁-C₆)-alkyl, a (C₂-C₆)-alkenyl, a (C₂-C₆)-alkynyl, a (C₆-C₁₂)-aryl, a 5 to 7-membered-ring heterocycle, a (C₁-C₆)-alkoxy, a (C₂-C₆)-acyl, a (C₁-C₆)-alcohol, a carboxylic group, a (C₂-C₆)-ester, a (C₁-C₆)-amine, an amino group, a (C₁-C₆)-amide, a (C₁-C₆)-imine, a (C₁-C₆)-nitrile, a hydroxyl, an aldehyde, a (C₁-C₆)-halogenoalkyl, a thiol, a (C₁-C₆)-thioalkyl, a (C₁-C₆)-sulfone, a (C₁-C₆)-sulfoxide group and a halogen atom;
R³ is selected from the group consisting of a (C₁-C₆)-alkyl, a (C₂-C₆)-alkenyl and a (C₂-C₆)-alkynyl and a (C₁-C₆)-amine group, optionally substituted by at least one substituent selected from a hydroxyl, a carboxylic group, an aldehyde, a halogen atom and a thiol; and a (C₆-C₁₂)-aryl and a 5 to 7-membered-ring heterocycle, optionally substituted by at least one substituent selected from the group consisting of a (C₁-C₆)-alkyl, a (C₁-C₆)-alkoxy group and a halogen atom; or any pharmaceutically acceptable salt thereof.

### Legends of the Figures

**Figure 1****:** Prokineticin-2 via PKR1 inhibits the differentiation of cEPPC into adipocytes. ***Fig. 1A*****.** Oil red staining after pro-adipogenic treatment (MIX), and pretreatment with prokineticin-2 with pro-adipogenic treatment (PK-2+MIX). ***Fig. 1B*****.** (*)Shows difference between MIX and PK-2+MIX treated wild type cEPPC (p<0.05). In PKR1-KO mice prokineticin-2 was not able to inhibit adipogenesis.
**Figure 2****:** Characterisation of adipogenic gene expression in cEPPC differentiating into adipocytes. Quantitative RT-PCR analysis of cEPPC treated by prokineticin-2 (PK-2), proadipogenic cocktail (MIX) or PK-2+MIX during 1 week or 2 weeks. (*) Shows difference between PK-2 and MIX treatment (p<0.05), (#) shows difference between MIX and PK-2+MIX.
**Figure 3****:** Prokineticin-2 treatment of the cEPPC induced mature, and spontaneously beating cardiomyocytes as beating frequence is increased by beta adrenergic stimuli (isoproterenol) (Fig. 3A and 3B) and as they are positively stained by troponin, a cardiomyocyte marker (Fig. 3C).

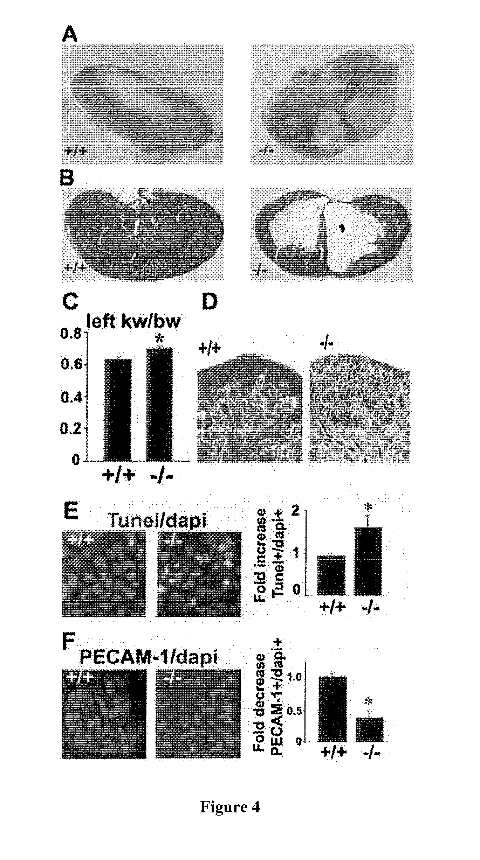

**Figure 4: *Fig. 4A***. Gross morphology of kidneys. ***Fig. 4B***. Mallory tetrachrome staining of cryosectioned kidneys. ***Fig. 4C***. Kidney weight to body weight ratio (n=8, p<0.05). ***Fig. 4D***. Paraffin-sectioned neonatal kidneys stained with Mallory tetrachrome. ***Fig. 4E****.* Representative illustration of TUNEL analyses on kidneys. The histogram and photographs show larger numbers of TUNEL-positive apoptotic cells in mutant glomeruli (n=4, p<0.05). ***Fig. 4F***. Representative illustration of PECAM-1-positive capillaries in glomeruli. The histogram shows that there were fewer PECAM-1-positive endothelial cells in mutant glomeruli (n=3, p<0.01).

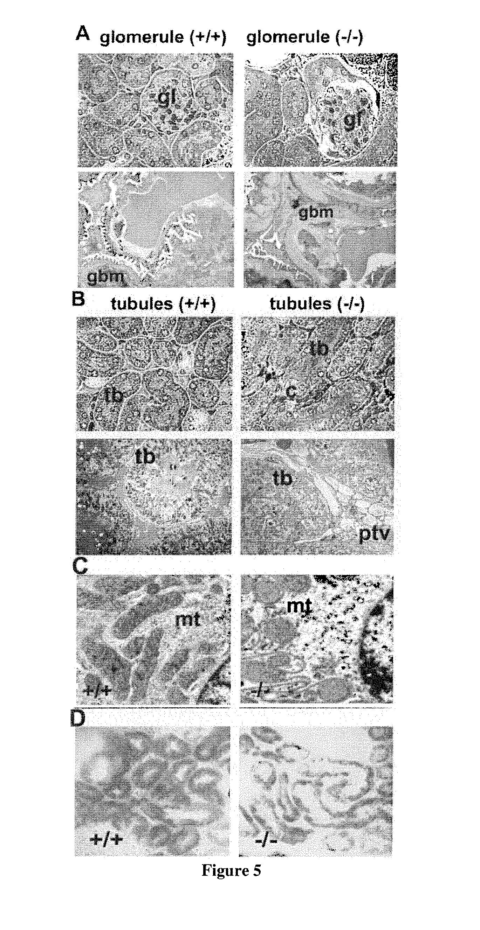

**Figure 5: *Fig. 5A***. Mallory tetrachrome staining on cryosectioned kidney samples. (Upper) Electron microscopy analyses on kidney samples. Thickening of the glomerular basement membrane (gbm) was evident in mutant kidneys (lower). ***Fig. 5B***. Histological analyses showed enlarged tubules (tb) and severe fibrosis between the tubules in mutant kidneys. Electron microscopy revealed disorganized tubular (tb) and dysmorphic peritubular vessels (ptv) and collagen (c) deposition in mutant kidneys. ***Fig. 5C***. Mutants had fewer mitochondria in their tubules, with fewer granulocyte and crista structures. ***Fig. 5D***. Representative mitochondrial function analyses based on SDH staining in cryosectioned kidneys.
**Figure 6****:** Urine and serum analyses (24-week-old mice, n=6 for each group)
**Figure 7****:** Expression of PKR1 in mouse kidney. Impaired glomerular endothelial cells (ec) with fenestration and podocyte structures (pod) in 36 weeks old mutant mice kidneys (-/-) demonstrated by electron microscopic analysis.

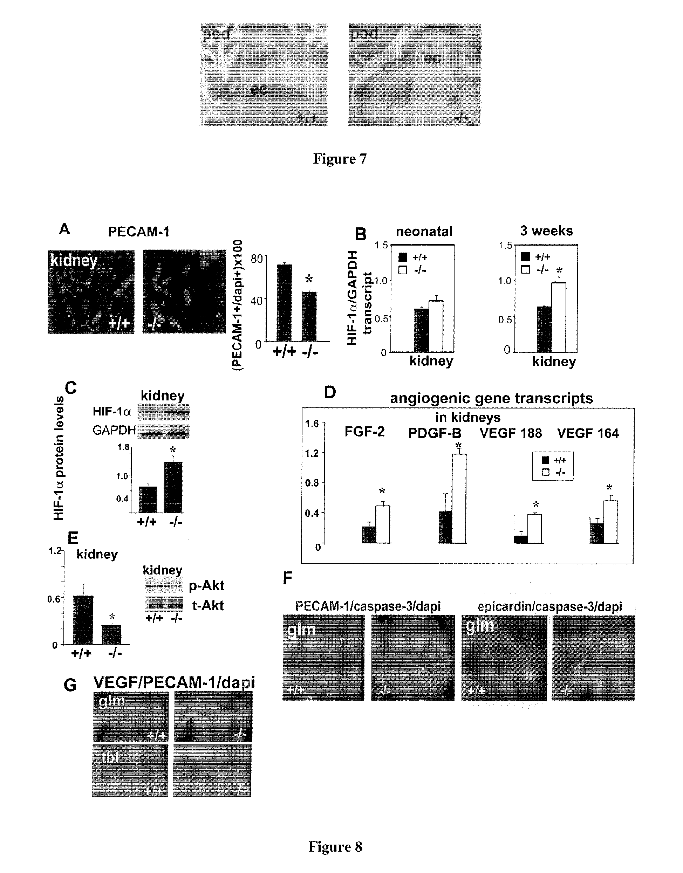

**Figure 8: *Fig. 8A*****.** Representative illustration of capillary density by an endothelial marker, PECAM-1 (n=3) staining in cryosectioned neonatal (P1) kidneys. Original magnification=X40. Quantification of capillary density calculated as numbers of PECAM-1 positive cells/dapi+ cells corrected with 100 per high power field. Histograms show the quantitative changes between wild type (+/+) and null mutants (-/-), n=4, p<0.05. ***Fig. 8B*****.** HIF-1α transcript levels in RNA derived from neonatal (left) and 3 weeks old kidneys (right, n=4, p<0.05). ***Fig. 8C*****.** Western blot analyses with HIF-1α and GAPDH antibodies on kidney protein confirm an increase in HIF-1α levels in 3 weeks old mutant kidneys (n=3, duplicated, p<0.05). ***Fig. 8D*****.** Histogram represents RT-PCR analysis showing that expression of pro-angiogenic factors, PDGF, FGF and VEGF were differently altered in mutant kidneys at the age of 3 weeks (n=4). ***Fig. 8E*****.** Western Blot analysis and histogram show diminished levels of phosphorylated-Akt form in neonatal mutant kidneys (n=4). ***Fig. 8F******.*** Co-immunostaining of kidney sections with active caspase-3 antibody, endothelium specific PECAM-1, progenitor cell specific epicardin antibodies in glomerulus. ***Fig. 8G*****.** VEGF immunostaining of glomerulus (glm) and tubules (tbl).

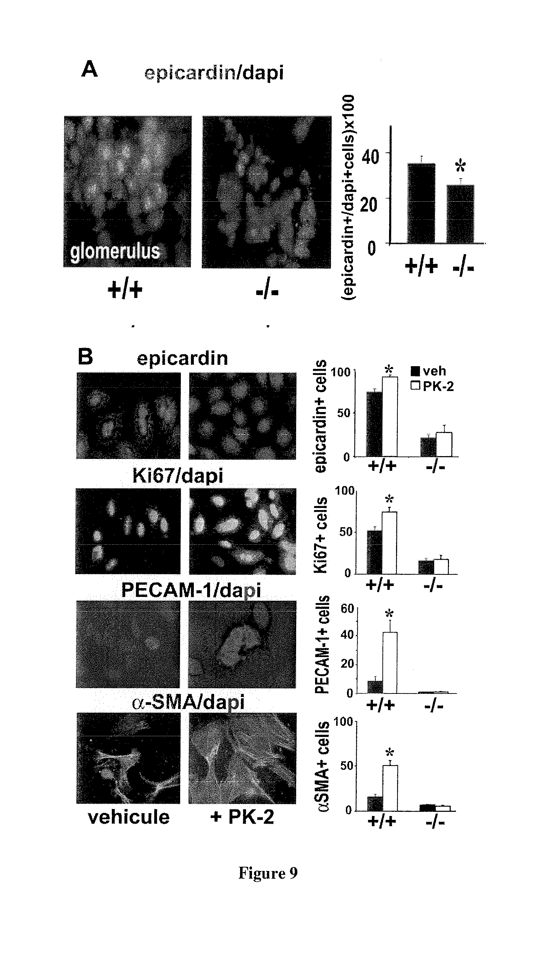

**Figure 9: *Fig. 9A*****.** Epicardin+ progenitor cells in glomeruli. Original magnification=X40. Quantification of epicardin positive cells were calculated as numbers of epicardin positive cells/dapi+ cells corrected with 100 per high power field (n=5, p<0.05). ***Fig. 9B*****.** Representative illustration of epicardin, Ki67, PECAM-1 and α-SMA positive cell number in the renal EPDCs derived from kidney explants treated with vehicle or prokineticin-2 (5 or 10 nM) for 24 h. Original magnification=X63. Histograms show the quantitative changes between wild type (+/+) and null mutants (-/-). Quantification of the specific cell number/dapi positive cell number corrected with 100 per high power field (n=4, p<0.05).
**Figure 10****:** Formulas of PKR1 agonists IS1 to IS12 and IS14 to IS19.
**Figure 11**: Synthesis of non peptide PKR1 agonists.
**Figure 12**: Induction of *in vitro* angiogenesis by prokineticin-2 and IS1 compound. ***Fig. 12A***: tube-like formation of the endothelial cells on Matrigel. ***Fig. 12B***: quantification of the tube-like formation of the endothelial cells on Matrigel.
**Figure 13****:** Induction of *in vitro* angiogenesis by prokineticin-2 and IS compounds. H5V cells were infected with adenovirus carrying PKR1 cDNA in order to dominantly express PKR1. *Fig. 13A**:* 1 µM prokineticin-2 or IS compound. *Fig. 13B*: 10 µM prokineticin-2 or IS compound.
Figure 14: intracellular calcium release detected by fura-4 labeled CHO utilizing confocal microscope analyses.
Figure 15: PKR1 internalization in CHO cells GFP-PKR1.

### Detailed description of the invention

Prokineticin-2, also called Bv8, and prokineticin-1, also called endocrine gland-derived vascular endothelial growth factor (EG-VEGF) are two secreted proteins from the AVIT secreted protein family that are widely express among mammalian tissues.

These molecules exert their biological functions through activation of two closely related G-protein-coupled receptors, the prokineticin receptors 1 and 2 (PKR1 and PKR2) (Lin et al., 2002; Masuda et al., 2002; Soga et al., 2002). Both PKR1 and PKR2 are mainly expressed in human and rat endocrine tissues, including thyroid, pituitary, and adrenal glands, as well as in the ovary and testis. PKR2 and to a lesser extent PKR1 are expressed in the brain. PKR1, originally called GPR73, is mainly expressed in spleen, prostrate, pancreas, monocytes leukocytes, and in human heart (Urayama et al., 2007).

In cardiomyocytes, prokineticin-2 via PKR1 protects cardiomyocytes against hypoxia induced apoptosis (Urayama et al., 2007). It was also shown that transient PKR1 gene transfer reduces mortality and preserves left ventricular function by promoting angiogenesis and cardiomyocyte survival in the coronary ligation mouse model for myocardial infarction (Urayama et al., 2007).

The inventors have herein demonstrated that prokineticin receptor 1 agonists, such as prokineticin-2, promote the differentiation of cardiac epicardin positive progenitor cells (cEPPC) into cardiomyocytes and, in the same time, block the differentiation of these cells into adipocytes. The inventors have also provided evidence that epicardin positive progenitor cells are present in kidney (rEPPC) and that prokineticin receptor 1 agonists, in particular prokineticin-2, induce the differentiation of these renal epicardin positive progenitor cells (rEPPC) into endothelial and vascular smooth muscle cells, thereby promoting renal angiogenesis.

In a first aspect, the present invention concerns a prokineticin receptor-1 agonist for use for promoting the differentiation of cardial epicardin+ progenitor cells (cEPPC) into cardiomyocytes, in particular in a subject affected with a cardiac disease, and/or the differentiation of renal epicardin+ progenitor cells (rEPPC) into vasculogenic cells and/or glomerular cells, in particular in a subject affected with a renal disease.

As used herein, the term "prokineticin receptor-1" or "PKR1" refers to a G-protein-coupled receptor that could be activated by the binding of prokineticin-1 or prokineticin-2. It may be also named GPR73 or PROKR1. The reference amino acid sequence for human PKR1 can be retrieved from the Genbank Accession No. NP_620414. The reference entry for human PKR1 in the transcriptome database UniGene is Hs.683430.

As used herein, the term "epicardin positive progenitor cells" or "epicardin+ progenitor cells" refers to progenitor cells expressing the transcription factor epicardin, also named capsulin, POD1 or TCF-21. Progenitor cells are undifferentiated cells with a limited capacity of self-renewal via a limited number of cell divisions. As used herein, the term "progenitor cells" refers to multipotent cells that can differentiate into only few cell types. In particular, cardiac epicardin+ progenitor cells have the ability to differentiate into cardiomyocytes, adipocytes and vasculogenic cells. Renal epicardin+ progenitor cells (rEPPC) have the ability to differentiate into glomerular cells, adipocytes and vasculogenic cells. The expression of epicardin may be assessed by any method known by the skilled person such as immunochemistry using an anti-epicardin antibody or quantitative RT-PCR. Cardiac and renal epicardin+ cells can be isolated from cardiac or renal tissue by any method known by the skilled person. For example, they can be isolated by using affinity binding with an anti-epicardin antibody, as illustrated in the experimental section. It has to be understood that progenitor cells are not embryonic stem cells, and in particular human embryonic stem cells.

As used herein, the term "patient" or "subject" refers to any mammal, preferably a human being.

The cardiac disease may be any cardiac disease inducing cardiomyocyte lesion, injury, death, necrosis or apoptosis. In a particular embodiment, the cardiac disease is selected from the group consisting of heart failure, myocardial infarction, ischemic heart disease, the cardiorenal syndrome and cardiovascular complications of diabetes. In a more particular embodiment, the cardiac disease is myocardial infarction or ischemic heart disease, preferably myocardial infarction.

The renal disease may be a renal disease inducing lesion, injury, death, necrosis or apoptosis of glomerular cells and/or an ischemic renal disease. In a particular embodiment, the renal disease is selected from the group consisting of chronic renal disease, renal artery stenosis, renal failure, acute kidney injury, acute-on-chronic renal failure, ischemic nephropathy, Churg-Strauss syndrome, Wegener's granulomatosis, the cardiorenal syndrome and renal complications of diabetes. In a more particular embodiment, the renal disease is acute kidney injury or ischemic nephropathy.

In a preferred embodiment, the subject is affected with a cardiorenal syndrome. This syndrome is a condition characterized by kidney failure and heart failure.

As used herein, the term "prokineticin receptor-1 agonist" or "PKR1 agonist" refers to any compound that is able to bind and activate the prokineticin receptor-1, as described above. The PKR1 agonist activity of a compound may be assessed by any method known by the skilled person. For example, the PKR1 agonist activity may be assessed by any of the methods disclosed in the experimental section: the measure of the capacity to induce in vitro angiogenesis, the measure of the capacity to activate the PKR1 signalling pathway (e.g. by measurement of the intracellular calcium), or the capacity to induce to internalization of the receptor (example 3). The agonist may be a peptide or a non peptidic compound.

In an embodiment, the PKR1 agonist is selected from the group consisting of prokineticin-2 (PK2) and prokineticin-1 (PK1), and an active fragment thereof, i.e. a fragment that is able to bind and activate PKR1. PK2 and PK1 may be used in their precursor or mature form. The NCBI accession number for the sequence of the human PK1 precursor is NP_115790.1. The NCBI accession number for the sequence of the human PK2 precursor is NP_001119600.1. In a particular embodiment, the PKR1 agonist is an active fragment of PK2. This fragment may be a peptide comprising 47 amino acids of the N-terminus of PK2 as described in the SEQ ID NO.: 1 and 2 of the international patent application WO 2005/097826.

In another embodiment, the PKR1 agonist is an antibody or a fragment thereof. As used herein, the term "antibody" is intended to refer broadly to any immunologic binding agent such as IgG, IgM, IgA, IgD and IgE, and humanized or chimeric antibody. In certain embodiments, IgG and/or IgM are preferred because they are the most common antibodies in the physiological situation and they are most easily manufactured. The term "a fragment thereof' is used to refer to any antibody-like molecule that has an antigen binding region, and includes antibody fragments such as Fab', Fab, F(ab') 2, single domain antibodies (DABs), Fv, scFv (single chain Fv), and the like. The techniques for preparing and using various antibody-based constructs and fragments are well known in the art. Means for preparing and characterizing antibodies are also well known in the art (See, e.g., Harlow and Lane, 1988). A "humanized" antibody is an antibody in which the constant and variable framework region of one or more human immunoglobulins is fused with the binding region, e.g. the CDR, of an animal immunoglobulin. "Humanized" antibodies contemplated in the present invention are chimeric antibodies from mouse, rat, or other species, bearing human constant and/or variable region domains, bispecific antibodies, recombinant and engineered antibodies and fragments thereof. Such humanized antibodies are designed to maintain the binding specificity of the non-human antibody from which the binding regions are derived, but to avoid an immune reaction against the non-human antibody. A "chimeric" antibody is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. The antibody may be, for example, an anti-mouse Bv8 monoclonal antibody (Genentech).

The peptidic agonist may comprise modifications inducing an improved resistance to proteolysis such as a -CONH- amide peptide bond replaced by a (CH₂-NH) reduced bond, a (NH-CO) retro-inverso bond, a (CH₂-O) methylene-oxy bond, a (CH₂-S) thiomethylene bond, a (CH₂-CH₂) carba bond, a (CO-CH₂) ceto-methylene bond, a (CHOH-CH₂) hydroxyethylene bond), a (N-N) bond, a E-alcene bond or a -CH=CH- bond. The peptidic agonist can also present either carboxylic (i.e. -COO⁻) or carboxyl-amidated (i.e. -CONH₂) C-terminal extremity.

In embodiments wherein the PKR1 agonist is a peptidic compound, the agonist may be administered to the subject in the form of a nucleic acid sequence encoding said agonist. The sequence encoding the peptidic agonist may be obtained by cloning, by PCR amplification or by chemical synthesis according to the conventional techniques in common use and using the literature data. The nucleic acid may be inserted in a recombinant vector allowing its expression in eukaryote cells. The structure and composition of such vectors is well-known by the skilled person. The expression vector may be a plasmid or a viral vector. In particular, it may be derived from a lentivirus, a poxvirus, an adenovirus, a retrovirus, a herpesvirus or an adenovirus-associated virus. In this vector, the encoding sequence is placed under the control of elements necessary for its expression in a eucaryote host cell or organim, and in particular in human. These elements include elements regulating transcription as well as signals for initiation and termination of translation. For instance, the promoter may be selected from the group consisting of SV40 (Simian Virus 40) promoter, the HMG (Hydroxy-Methyl-Glutaryl-coenzyme A) promoter, the TK (Thymidine Kinase) promoter, the CMV (cytomegalovirus) promoter, the RSV (Rous Sarcoma Virus) promoter and the MLP promoter (Major Late Promoter).

In a preferred embodiment, the PKR1 agonist is a non peptide compound. In particular, the PKR1 agonist is a compound of formula (I) 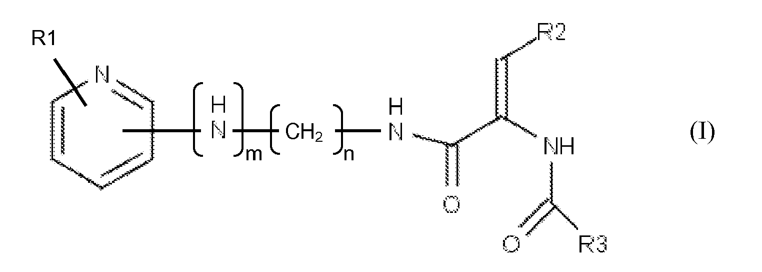 wherein
m is selected from 0 and 1;
n is selected from 0, 1 and 2;
R¹ is selected from the group consisting of a hydrogen atom; a halogen atom; a (C₁-C₆)-alkyl, a (C₂-C₆)-alkenyl and a (C₂-C₆)-alkynyl group, optionally substituted by at least one substituent selected from a hydroxyl, a carboxylic group, an aldehyde, a halogen atom and a thiol; a (C₆-C₁₂)-aryl; a 5 to 7-membered-ring heterocycle; a (C₁-C₆)-alkoxy, a (C₂-C₆)-acyl, a (C₂-C₆)-ester, a (C₁-C₆)-amine, a (C₁-C₆)-amide, a (C₁-C₆)-imine, a (C₁-C₆)-nitrile, a (C₁-C₆)-thioalkyl, a (C₁-C₆)-sulfone and a (C₁-C₆)-sulfoxide group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;
R² is selected from the group consisting of a (C₆-C₁₂)-aryl group and a 5 to 7-membered-ring heterocycle, preferably from phenyl and furan, optionally substituted by at least one substituent selected from the group consisting of a (C₁-C₆)-alkyl, a (C₂-C₆)-alkenyl, a (C₂-C₆)-alkynyl, a (C₆-C₁₂)-aryl, a 5 to 7-membered-ring heterocycle, a (C₁-C₆)-alkoxy, a (C₂-C₆)-acyl, a (C₁-C₆)-alcohol, a carboxylic group, a (C₂-C₆)-ester, a (C₁-C₆)-amine, an amino group, a (C₁-C₆)-amide, a (C₁-C₆)-imine, a (C₁-C₆)-nitrile, a hydroxyl, an aldehyde, a (C₁-C₆)-halogenoalkyl, a thiol, a (C₁-C₆)-thioalkyl, a (C₁-C₆)-sulfone, a (C₁-C₆)-sulfoxide group and a halogen atom;
R³ is selected from the group consisting of a (C₁-C₆)-alkyl, a (C₂-C₆)-alkenyl, a (C₂-C₆)-alkynyl and a (C₁-C₆)-amine group, optionally substituted by at least one substituent selected from a hydroxyl, a carboxylic group, an aldehyde, a halogen atom and a thiol; and a (C₆-C₁₂)-aryl and a 5 to 7-membered-ring heterocycle, optionally substituted by at least one substituent selected from the group consisting of a (C₁-C₆)-alkyl, a (C₁-C₆)-alkoxy group and a halogen atom;
or any pharmaceutically acceptable salt thereof.

As used herein, the term "alkyl" refers to a univalent radical containing only carbon and hydrogen atoms arranged in a chain. The alkyl group may be linear or branched. More specifically, the term "alkyl" means a group such as methyl, ethyl, propyl, isopropyl, butyl, isobutyl, tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl and the other isomeric forms thereof. (C₁-C₆)-alkyl includes methyl, ethyl, propyl, isopropyl, butyl, isobutyl, tert-butyl, pentyl, hexyl and the other isomeric forms thereof. (C₁-C₃)-alkyl includes methyl, ethyl, propyl, or isopropyl. The term "Me" as used herein, refers to a methyl group. The term "Et" as used herein, refers to an ethyl group.

As used herein, the term "alkoxy" corresponds to an alkyl group bonded to the molecule by an -O- (ether) bond. (C₁-C₆)- alkoxy includes methoxy, ethoxy, propyloxy, butyloxy, pentyloxy, hexyloxy and the other isomeric forms thereof. (C₁-C₃)-alkoxy includes methoxy, ethoxy, propyloxy, and isopropyloxy.

The term "alkenyl" refers to an alkyl group having at least one unsaturated ethylene bond and the term "alkynyl" refers to an alkyl group having at least one unsaturated acetylene bond. (C₂-C₆)-alkenyl includes ethenyl, propenyl (1-propenyl or 2-propenyl), 1-or 2- methylpropenyl, butenyl (1-butenyl, 2-butenyl, or 3-butenyl), methylbutenyl, 2-ethylpropenyl, pentenyl (1-pentenyl, 2-pentenyl, 3-pentenyl, 4-pentenyl), hexenyl (1-hexenyl, 2-hexenyl, 3-hexenyl, 4-hexenyl, 5-hexenyl), and the other isomeric forms thereof. (C₂-C₃)-alkenyl includes ethenyl, 1-propenyl and 2-propenyl. (C₂-C₆)-alkynyl includes ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, or 5-hexynyl and the other isomeric forms thereof. (C₂-C₃)-alkynyl includes ethynyl, 1-propynyl and 2-propynyl.

The "aryl" groups are monocyclic, bicyclic, or polycyclic aromatic hydrocarbons. Examples include phenyl, biphenyl, α-naphthyl, β-naphthyl, anthracenyl, phenanthrenyl, fluorenyl, indenyl, pentalenyl, azulenyl, biphenylenyl and the like. This term is also intended to include the partially hydrogenated derivatives of the aromatic hydrocarbons groups enumerated above. The aryl groups of the present invention can be substituted or unsubstituted. (C₆-C₁₂)-aryl includes phenyl, biphenyl, indenyl, pentalenyl and azulenyl.

The term "heterocycle", as used herein, refers to 5-10 membered heterocyclic ring systems comprising one or more heteroatoms, preferably 1 to 5 endocyclic heteroatoms. They may be mono-, bi- or tri-cyclic systems. They may be aromatic or not. Examples of aromatic heterocycles include pyridine, pyridazine, pyrimidine, pyrazine, furan, thiophene, pyrrole, oxazole, thiazole, isothiazole, imidazole, pyrazole, oxadiazole, triazole, thiadiazole and triazine groups. Examples of non-aromatic heterocycles include piperazine and piperidine. Examples of 5 to 7-membered-ring heterocycles include pyrrolidine, tetrahydrofuran, tetrahydrothiophene, pyrrole, furan, thiophene, piperidine, tetrahydropyran, thiane, pyridine, pyrylium, thiopyran, azepane, oxepane, thiepane, azepine, oxepine and thiepine.

The term "heteroatom" refers to any atom that is not carbon or hydrogen. In particular embodiments, this term refers to N, S, or O.

"Acyl" groups correspond to alkyl groups bonded to the molecule by a -CO-(carbonyl) group. (C₂-C₆)-acyl includes acetyl, propylacyl, butylacyl, pentylacyl, hexylacyl and the other isomeric forms thereof. (C₂-C₃)-acyl includes acetyl, propylacyl and isopropylacyl.

"Alcohol" groups correspond to alkyl groups containing at least one hydroxyl group. Alcohol can be primary, secondary or tertiary. (C₁-C₆)-alcohol includes methanol, ethanol, propanol, butanol, pentanol, hexanol and the other isomeric forms thereof. (C₁-C₃)-alcohol includes methanol, ethanol, propanol and isopropanol.

"Ester" groups correspond to alkyl groups bonded to the molecule by a -COO- (ester) bond. (C₂-C₆)-ester includes methylester, ethylester, propylester, butylester, pentylester and the other isomeric forms thereof. (C₂-C₃)-ester includes methylester and ethylester.

"Amine" groups correspond to alkyl groups bonded to the molecule by a -N- (amine) bond. (C₁-C₆)-amine includes methylamine, ethylamine, propylamine, butylamine, pentylamine, hexylamine and the other isomeric forms thereof. (C₁-C₃)-amine includes methylamine, ethylamine, and propylamine.

"Amide" groups correspond to alkyl groups containing at least one carbonyl (CO) group bound to a nitrogen atom. (C₁-C₆)-amide includes methylamide, ehtylamide, propylamide, butylamide, pentylamide, hexylamide and the other isomeric forms thereof. (C₁-C₃)-amide includes methylamide, ehtylamide and propylamide.

"Imine" groups correspond to alkyl groups having a (-C=N-) bond. (C₁-C₆)-imine includes methylimine, ethylimine, propylimine, butylimine, pentylimine, hexylimine and the other isomeric forms thereof. (C₁-C₃)-imine includes methylimine, ethylimine, and propylimine.

"Nitrile" groups correspond to alkyl groups having a (-C≡N-) bond.

The halogen can be Cl, Br, I, or F, preferably Cl, Br or F, more preferably Cl.

"Halogenoalkyl" groups correspond to alkyl groups having at least one halogen. The groups can be monohalogenated or polyhalogenated, containing the same or different halogen atoms. For example, the group can be an trifluoroalkyl (CF₃-R). (C₁-C₆)-halogenoalkyl includes halogenomethyl, halogenoethyl, halogenopropyl, halogenobutyl, halogenopentyl, halogenohexyl and the other isomeric forms thereof. (C₁-C₃)-halogenoalkyl includes halogenomethyl, halogenoethyl, and halogenopropyl.

"Thioalkyl" groups correspond to alkyl groups bonded to the molecule by a -S-(thioether) bond. (C₁-C₆)-thioalkyl includes thiomethyl, thioethyl, thiopropyl, thiobutyl, thiopentyl, thiohexyl and the other isomeric forms thereof. (C₁-C₃)-thioalkyl includes thiomethyl, thioethyl, and thiopropyl.

"Sulfone" groups correspond to alkyl groups bonded to the molecule by a -SOO-(sulfone) bond. (C₁-C₆)-sulfone includes methylsulfone, ethylsulfone, propylsulfone, butylsulfone, pentylsulfone, hexylsulfone and the other isomeric forms thereof. (C₁-C₃)-sulfone includes methylsulfone, ethylsulfone and propylsulfone.

"Sulfoxide" groups correspond to alkyl groups bonded to the molecule by a -SO-(sulfoxide) group. (C₁-C₆) sulfoxide includes methylsulfoxide, ethylsulfoxide, propylsulfoxide, butylsulfoxide, pentylsulfoxide, hexylsulfoxide and the other isomeric forms thereof. (C₁-C₃) sulfoxide includes methylsulfoxide, ethylsulfoxide, propylsulfoxide and isopropylsulfoxide.

The term "pharmaceutically acceptable salt" refers to salts which are non-toxic for a patient and suitable for maintaining the stability of a therapeutic agent and allowing the delivery of said agent to target cells or tissue. Pharmaceutically acceptable salts are well known in the art (Berge et al., 1977). These salts can be prepared in situ during the final isolation and purification of the agonists of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, 3-phenylpropionate, phosphate, picrate, pivalate, propionate, stearate, succinate, sulfate, tartrate, thiocyanate, p-toluenesulfonate, undecanoate, valerate salts, and the like.

In particular, the PKR1 agonist of formula (I) is defined as above, with the proviso that when m is 0, n is 1, R¹ is a hydrogen atom and R² is a furan group, then R³ is not a phenyl group or a phenyl group substituted in para by a methoxy group; and when m is 0, n is 0, R¹ is a hydrogen atom and R² is a furan group, then R³ is not a phenyl group.

### In an embodiment,

R¹ is selected from the group consisting of a hydrogen atom; a halogen atom; a (C₁-C₃)-alkyl, a (C₂-C₃)-alkenyl and a (C₂-C₃)-alkynyl group, optionally substituted by at least one substituent selected from a hydroxyl, a carboxylic group, an aldehyde, a halogen atom and a thiol; a (C₆-C₁₂)-aryl, preferably a phenyl group; a 5 to 7-membered-ring heterocycle; a (C₁-C₃)-alkoxy, a (C₂-C₃)-acyl, a (C₂-C₃)-ester, a (C₁-C₃)-amine, a (C₁-C₃)-amide, a (C₁-C₃)-imine, a (C₁-C₃)-nitrile, a (C₁-C₃)-thioalkyl, a (C₁-C₃)-sulfone and a (C₁-C₃)-sulfoxide group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;

R² is selected from the group consisting of a phenyl group and a 5 to 7-membered-ring N-, O- or S-heterocycle, optionally substituted by at least one substituent selected from the group consisting of a (C₁-C₃)-alkyl, a (C₂-C₃)-alkenyl, a (C₂-C₃)-alkynyl, a phenyl group, a 5-membered-ring heterocycle, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a (C₁-C₃)-imine, a (C₁-C₃)-nitrile, a hydroxyl, an aldehyde, a (C₁-C₃)-halogenoalkyl, a thiol, a (C₁-C₃)-thioalkyl, a (C₁-C₃)-sulfone, a (C₁-C₃)-sulfoxide group and a halogen atom;

R³ is selected from the group consisting of a (C₁-C₆)-alkyl, a (C₂-C₃)-alkenyl, a (C₁-C3)-amine and a (C₂-C₃)-alkynyl group, optionally substituted by at least one substituent selected from a hydroxyl, a carboxylic group, an aldehyde, a halogen atom and a thiol; and a phenyl group and a 5 to 7-membered-ring heterocycle, optionally substituted by at least one substituent selected from the group consisting of a (C₁-C₃)-alkyl, a (C₁-C₃)-alkoxy group and a halogen atom.

In an embodiment, R¹ is selected from the group consisting of a hydrogen atom; a halogen atom; a (C₁-C₃)-alkyl, optionally substituted by at least one substituent selected from a hydroxyl, a carboxylic group, an aldehyde, a halogen atom and a thiol; a (C₁-C₃)-alkoxy, a (C₂-C₃)-acyl, a (C₂-C₃)-ester, a (C₁-C₃)-amine, a (C₁-C₃)-amide and a (C₁-C₃)-thioalkyl, group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol.

In a particular embodiment, R¹ is selected from the group consisting of a hydrogen atom, a halogen atom, a (C₁-C₃)-alkyl group and a (C₁-C₃)-alkoxy group, said (C₁-C₃)-alkyl group and/or (C₁-C₃)-alkoxy group being optionally substituted by at least one substituent selected from an hydroxyl, a carboxylic group, an aldehyde, a halogen atom and a thiol.

In a more particular embodiment, R¹ is selected from the group consisting of a hydrogen atom, a halogen atom, a (C₁-C₃)-alkyl group and a (C₁-C₃)-alkoxy group. Preferably, R¹ is selected from the group consisting of a hydrogen atom, a halogen atom, a methyl group and a methoxy group. More preferably, R¹ is a hydrogen atom.

In an embodiment, R² is selected from the group consisting of a phenyl group and a 5 to 7-membered-ring N-, O- or S-heterocycle, preferably a 5-membered-ring N-, O- or S-heterocycle, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom.

In a particular embodiment, R² is selected from the group consisting of a phenyl, a pyrrole, a furan and a thiophene group, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom.

In a more particular embodiment, R² is selected from the group consisting of a phenyl, a pyrrole, a furan and a thiophene group, optionally substituted by at least one substituent selected from the group consisting of a (C₁-C₃)-alkyl and a (C₁-C₃)-alkoxy group, preferably methyl and methoxy. Preferably, R² is selected from the group consisting of a phenyl group, optionally substituted by a methoxy, a furan and a thiophene group.

In an embodiment, R³ is selected from the group consisting of a (C₁-C₆)-alkyl and a (C₁-C₆)-amine group optionally substituted by at least one substituent selected from a hydroxyl, a carboxylic group, an aldehyde, a halogen atom and a thiol; and a phenyl group and a 5 to 7-membered-ring N-, O- or S-heterocycle, optionally substituted by at least one substituent selected from the group consisting of a (C₁-C₃)-alkyl, a (C₁-C₃)-alkoxy group and a halogen atom.

In a particular embodiment, R³ is selected from the group consisting of a methyl, an ethyl, a propyl, an isopropyl, a butyl, an isobutyl, a tert-butyl group; a phenyl, a pyrrole, a furan and a thiophene group, optionally substituted by at least one substituent selected from the group consisting of a (C₁-C₃)-alkyl group, preferably methyl, a (C₁-C₃)-alkoxy group, preferably methoxy, and a halogen atom, preferably chlorine.

In a more particular embodiment, R³ is selected from the group consisting of a methyl, an ethyl, a propyl, an isopropyl, a butyl, an isobutyl, a tert-butyl group; and a phenyl group, optionally substituted by at least one substituent selected from the group consisting of a (C₁-C₃)-alkyl group, preferably methyl, a (C₁-C₃)-alkoxy group, preferably methoxy, and a halogen atom, preferably chlorine. Preferably, R³ is selected from the group consisting of a tert-butyl group and a phenyl group, optionally substituted by at least one substituent selected from the group consisting of a methyl group, a methoxy group and chlorine. More preferably, R³ is selected from the group consisting of a tert-butyl, a phenyl, a methoxyphenyl and a chlorophenyl group.

In an embodiment, R¹ is selected from the group consisting of a hydrogen atom; a halogen atom; a (C₁-C₃)-alkyl, optionally substituted by at least one substituent selected from a hydroxyl, a carboxylic group, an aldehyde, a halogen atom and a thiol; a (C₁-C₃)-alkoxy, a (C₂-C₃)-acyl, a (C₂-C₃)-ester, a (C₁-C₃)-amine, a (C₁-C₃)-amide and a (C₁-C₃)-thioalkyl, group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;

R² is selected from the group consisting of a phenyl group and a 5 to 7-membered-ring N-, O- or S-heterocycle, preferably a 5-membered-ring N-, O- or S-heterocycle, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom; and

R³ is selected from the group consisting of a (C₁-C₆)-alkyl and a (C₁-C₆)-amine group, optionally substituted by at least one substituent selected from a hydroxyl, a carboxylic group, an aldehyde, a halogen atom and a thiol; and a phenyl group and a 5 to 7-membered-ring N-, O- or S-heterocycle, optionally substituted by at least one substituent selected from the group consisting of a (C₁-C₃)-alkyl, a (C₁-C₃)-alkoxy group and a halogen atom.

### In a particular embodiment,

R¹ is selected from the group consisting of a hydrogen atom, a halogen atom, a (C₁-C₃)-alkyl group and a (C₁-C₃)-alkoxy group, said (C₁-C₃)-alkyl group and/or (C₁-C₃)-alkoxy group being optionally substituted by at least one substituent selected from an hydroxyl, a carboxylic group, an aldehyde, a halogen atom and a thiol;

R² is selected from the group consisting of a phenyl, a pyrrole, a furan and a thiophene group, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom; and

R³ is selected from the group consisting of a methyl, an ethyl, a propyl, an isopropyl, a butyl, an isobutyl, a tert-butyl group; a phenyl, a pyrrole, a furan and a thiophene group, optionally substituted by at least one substituent selected from the group consisting of a (C₁-C₃)-alkyl group, preferably methyl, a (C₁-C₃)-alkoxy group, preferably methoxy, and a halogen atom, preferably chlorine.

### In a more particular embodiment,

R¹ is selected from the group consisting of a hydrogen atom, a halogen atom, a (C₁-C₃)-alkyl group and a (C₁-C₃)-alkoxy group, preferably from the group consisting of a hydrogen atom, a halogen atom, a methyl group and a methoxy group, and more preferably is a hydrogen atom;

R² is selected from the group consisting of a phenyl, a pyrrole, a furan and a thiophene group, optionally substituted by at least one substituent selected from the group consisting of a (C₁-C₃)-alkyl and a (C₁-C₃)-alkoxy, preferably from the group consisting of a phenyl group, optionally substituted by a methoxy, a furan and a thiophene group; and

R³ is selected from the group consisting of a methyl, an ethyl, a propyl, an isopropyl, a butyl, an isobutyl, a tert-butyl group; and a phenyl group, optionally substituted by at least one substituent selected from the group consisting of a (C₁-C₃)-alkyl group, a (C₁-C₃)-alkoxy group and a halogen atom, preferably from the group consisting of a tert-butyl, a phenyl, a methoxyphenyl and a chlorophenyl group.

In an embodiment, the PKR1 agonist has the formula (II) 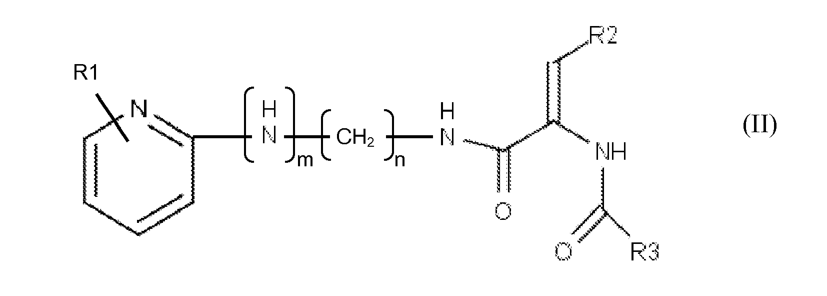 m, n, R¹, R² and R³ being as defined in formula (I) and in the above disclosed embodiments.

In another embodiment, the PKR1 agonist has the formula (III) 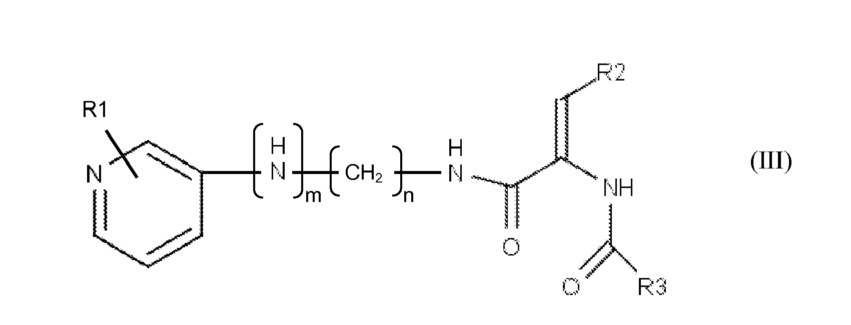 m, n, R¹, R² and R³ being as defined in formula (I) and in the above disclosed embodiments.

In another embodiment, the PKR1 agonist has the formula (IV) 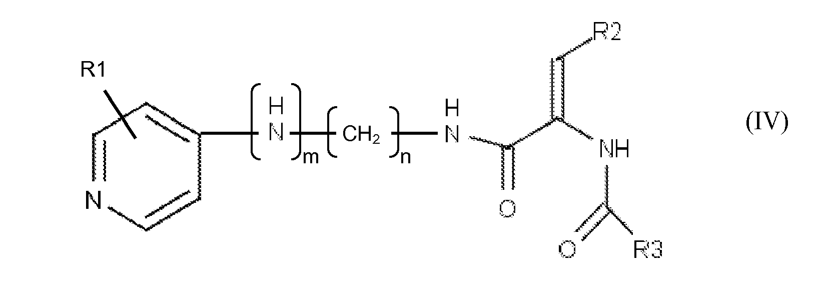 m, n, R¹, R² and R³ being as defined in formula (I) and in the above disclosed embodiments.

In an embodiment, the PKR¹ agonist has the formula (I), (II), (III) or (IV) and R¹ is a hydrogen atom; R² is selected from the group consisting of a 5-membered-ring heterocycle and a phenyl group, preferably from a furan, a thiophene and a phenyl group, optionally substituted by a (C₁-C₃)-alkoxy group, preferably a methoxy group; and R³ is selected from the group consisting of a (C₁-C₆)-alkoxy, preferably a tert-butyl group, and a phenyl group, optionally substituted by a substituent selected from the group consisting of a (C₁-C₃)-alkoxy group and a halogen atom, preferably from a methoxy group and chlorine.

In another embodiment, the PKR1 agonist has the formula (I), (II), (III) or (IV), and R¹ is a hydrogen atom; R² is selected from the group consisting of a 5-membered-ring heterocycle and a phenyl group, preferably from a furan, a thiophene and a phenyl group, optionally substituted by a (C₁-C₃)-alkoxy group, preferably a methoxy group; and R³ is a phenyl group. Preferably, in this embodiment, the PKR1 agonist has the formula (II).

In a particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group. Preferably, in this embodiment, the PKR1 agonist has the formula (II).

In another particular embodiment, the PKR1 agonist has the formula (I), (II), (III) or (IV), and m is 1; n is selected from 0 and 2; R¹ is a hydrogen atom; R² is selected from the group consisting of a 5-membered-ring 0- or S-heterocycle and a phenyl group, preferably from a furan, a thiophene and a phenyl group, optionally substituted by a (C₁-C₃)-alkoxy group, preferably a methoxy group; and R³ is a phenyl group. Preferably, in this embodiment, the PKR1 agonist has the formula (II).

In another particular embodiment, the PKR1 agonist has the formula (I), (II), (III) or (IV), and m is 1; n is 2; R¹ is a hydrogen atom; R² is selected from the group consisting of a 5-membered-ring O- or S-heterocycle and a phenyl group, preferably from a furan, a thiophene and a phenyl group, optionally substituted by a (C₁-C₃)-alkoxy group, preferably a methoxy group; and R³ is a phenyl group. Preferably, in this embodiment, the PKR1 agonist has the formula (II).

In another embodiment, the PKR1 agonist has the formula (I), (II), (III) or (IV), and m is 0; n is selected from 0, 1 and 2; R¹ is a hydrogen atom; R² is a 5-membered-ring O-heterocycle, preferably a furan group; and R³ is selected from the group consisting of a phenyl and a (C₁-C₆)-alkyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (III).

In a particular embodiment, the PKR1 agonist has the formula (I), (II), (III) or (IV), m is 0; n is selected from 1 and 2; R¹ is a hydrogen atom; R² is a 5-membered-ring O-heterocycle, preferably a furan group; and R³ is selected from the group consisting of a phenyl and a (C₁-C₆)-alkyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (III).

In another particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (III).

In another particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group. Preferably, in this embodiment, the PKR1 agonist has the formula (III).

In another particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group substituted by a (C₁-C₃)-alkoxy group, preferably a methoxy group. Preferably, in this embodiment, the PKR1 agonist has the formula (III).

In another particular embodiment, the PKR1 agonist has the formula (I), (II), (III) or (IV), and m is 0; n is selected from 1 and 2; R¹ is a hydrogen atom; R² is a 5-membered-ring O-heterocycle, preferably a furan group; and R³ is a phenyl group substituted by a halogen atom, preferably chlorine. Preferably, in this embodiment, the PKR1 agonist has the formula (III).

In another particular embodiment, the PKR1 agonist has the formula (I), (II), (III) or (IV), m is 0; n is selected from 1 and 2; R¹ is a hydrogen atom; R² is a 5-membered-ring O-heterocycle, preferably a furan group; and R³ is a (C₁-C₆)-alkyl group, preferably a tert-butyl group. Preferably, in this embodiment, the PKR1 agonist has the formula (III).

In another embodiment, the PKR1 agonist has the formula (I), (II), (III) or (IV), and m is 0; n is selected from 0 and 1; R¹ is a hydrogen atom; R² is a 5-membered-ring O-heterocycle, preferably a furan group; and R³ is a phenyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (IV).

In a particular embodiment, the PKR1 agonist has the formula (I), (II), (III) or (IV), m is 0; n is 1; R¹ is a hydrogen atom; R² is a 5-membered-ring O-heterocycle, preferably a furan group; and R³ is a phenyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (IV).

In another particular embodiment, the PKR1 agonist has the formula (I), (II), (III) or (IV), and m is 0; n is 1; R¹ is a hydrogen atom; R² is a 5-membered-ring O-heterocycle, preferably a furan group; and R³ is a phenyl group. Preferably, in this embodiment, the PKR1 agonist has the formula (IV).

In another particular embodiment, the PKR1 agonist has the formula (I), (II), (III) or (IV), and m is 0; n is 0; R¹ is a hydrogen atom; R² is a 5-membered-ring O-heterocycle, preferably a furan group; and R³ is a phenyl group, optionally substituted by at least one substituent selected from the group consisting of a (C₁-C₃)-alkoxy group and a halogen atom, preferably from methoxy and chlorine. Preferably, in this embodiment, the PKR1 agonist has the formula (IV).

In another particular embodiment, the PKR1 agonist has the formula (I), (II), (III) or (IV), and m is 0; n is 0; R¹ is a hydrogen atom; R² is a 5 to 7-membered-ring O-heterocycle, preferably a furan group; and R³ is a phenyl group. Preferably, in this embodiment, the PKR1 agonist has the formula (IV).

In an embodiment, the PKR1 agonist has the formula (II) or (IV), m is selected from 0 and 1; n is selected from 0, 1 and 2; R¹ is a hydrogen atom; R² is selected from the group consisting of a furan group and a phenyl group, optionally substituted by a methoxy group; and R³ is a phenyl group.

In a particular embodiment, the PKR1 agonist has the formula (II) or (IV), m is selected from 0 and 1; n is selected from 0, 1 and 2; R¹ is a hydrogen atom; R² is selected from the group consisting of a furan group and a phenyl group substituted by a methoxy group; and R³ is a phenyl group.

In an embodiment, the PKR1 agonist of formula (I), (II), (III) or (IV) has one or several of the following features: a) m is 0; b) n is 1; c) R¹ is a hydrogen atom; d) R² is a furan group; and e) R³ is a phenyl group. In another embodiment, the PKR1 agonist of formula (I), (II), (III) or (IV) has one or several of the following features: a) m is 1; b) n is 2; c) R¹ is a hydrogen atom; d) R² is a phenyl group; and e) R³ is a phenyl group. In another embodiment, the PKR1 agonist of formula (I), (II), (III) or (IV) has one or several of the following features: a) m is 1; b) n is 2; c) R¹ is a hydrogen atom; d) R² is a phenyl group substituted by a (C₁-C₃)-alkoxy group, preferably methoxy; and e) R³ is a phenyl group. In another embodiment, the PKR1 agonist of formula (I), (II), (III) or (IV) has one or several of the following features: a) m is 0; b) n is 1; c) R¹ is a hydrogen atom; d) R² is a furan group; and e) R³ is a phenyl group. In another embodiment, the PKR1 agonist of formula (I), (II), (III) or (IV) has one or several of the following features: a) m is 0; b) n is 1; c) R¹ is a hydrogen atom; d) R² is a furan group; and e) R³ is a phenyl group substituted by a halogen atom, preferably chlorine. In another embodiment, the PKR1 agonist of formula (I), (II), (III) or (IV) has one or several of the following features: a) m is 0; b) n is 1; c) R¹ is a hydrogen atom; d) R² is a furan group; and e) R³ is a phenyl group substituted by a (C₁-C₃)-alkoxy group, preferably methoxy. In another embodiment, the PKR1 agonist of formula (I), (II), (III) or (IV) has one or several of the following features: a) m is 0; b) n is 1; c) R¹ is a hydrogen atom; d) R² is a furan group; and e) R³ is a (C₁-C₆)-alkyl group, preferably a tert-butyl group. In an embodiment, the PKR1 agonist of formula (IV) has one or several of the following features: a) m is 0; b) n is 1; c) R¹ is a hydrogen atom; d) R² is a furan group; and e) R³ is a phenyl group.

The PKR1 agonist of formula (I), (II), (III) or (IV) may meet one of the above-listed features. It may also meet two of these features, for instance a) and b); a) and c); a) and d); a) and e); b) and c); b) and d); b) and e); c) and d); c) and e); or d) and e). The PKR1 agonist may meet three of these features, for instance a), b) and c); a), b) and d); a), b) and e); a), c) and d); a), c) and e); a), d) and e); b), c) and d); b), c) and e); b), d) and e); or c), d) and e). The PKR1 agonist may meet four of these features, for instance a), b), c) and d); a), b), c) and e); a), b), d) and e); b), c), d) and e); or a), c), d) and e). The PKR1 agonist may also meet all of these features, i.e. a), b), c), d) and e).

The PKR1 agonist may be selected from the group consisting of

| Name of agonist | Formula |
|---|---|
| IS1 | 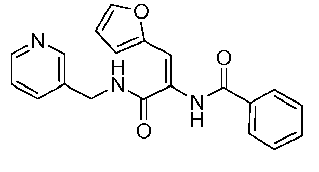 |
| IS2 | 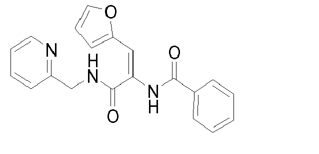 |
| IS3 | 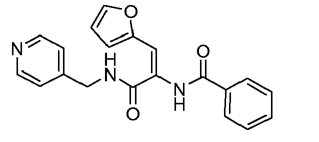 |
| IS4 | 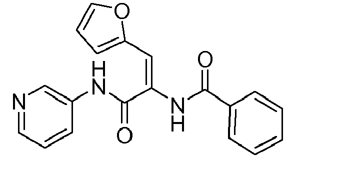 |
| IS5 | 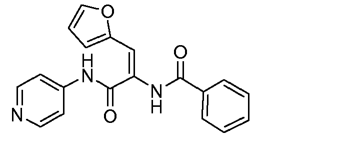 |
| IS6 | 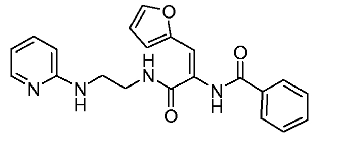 |
| IS7 | 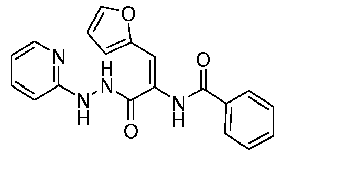 |
| IS8 | 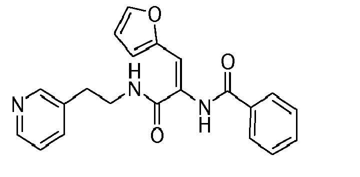 |
| IS9 | 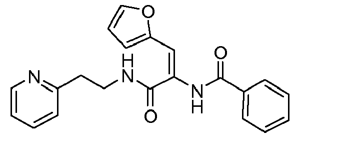 |
| IS10 | 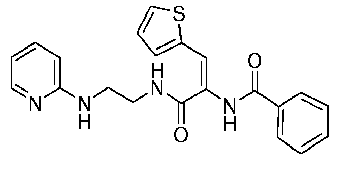 |
| IS11 | 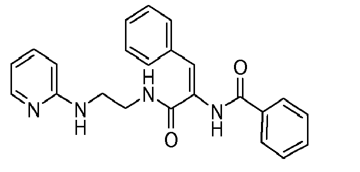 |
| IS12 | 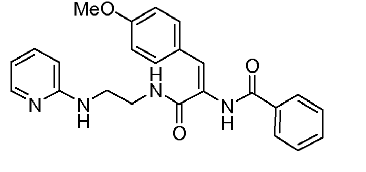 |
| IS14 | 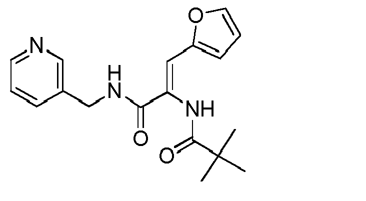 |
| IS15 | 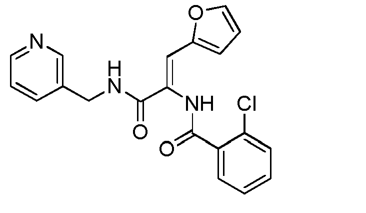 |
| IS16 | 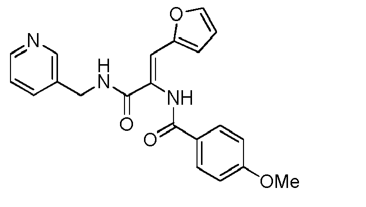 |
| IS17 | 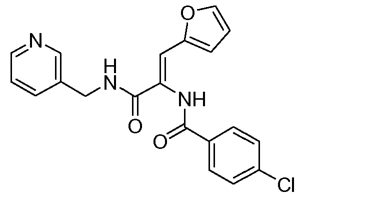 |
| IS18 | 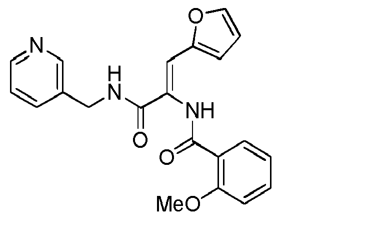 |
| IS19 | 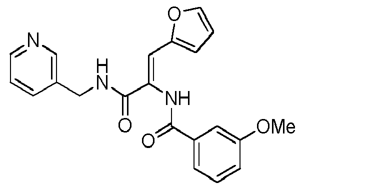 |

Preferably, the PKR1 agonist is selected from the group consisting of IS1, IS3, IS5, IS6, IS11, IS12, IS14, IS15, IS17 and IS18.

In a particular embodiment, the PKR1 agonist has the formula (I), (II), (III) or (IV), m, n, R¹, R² and R³ are defined as in formula (I) and in the above disclosed embodiments, with the proviso that when m is 0, n is 0, R¹ is a hydrogen atom, R² is a furan group, R³ is a phenyl group then the agonist has the formula (II) or (IV).

In a particular embodiment, the PKR1 agonist has the formula (I), (II), (III) or (IV), wherein m, n, R¹, R² and R³ have the same meaning as in the above disclosed embodiments, with the proviso that when m is 0, n is 1, R¹ is a hydrogen atom and R² is a furan group, then R³ is not a phenyl group or a phenyl group substituted in para by a methoxy group; and when m is 0, n is 0, R¹ is a hydrogen atom and R² is a furan group, then R³ is not a phenyl group.

In a particular embodiment, the PKR¹ agonist has the formula (I), (II), (III) or (IV), wherein n, R¹, R² and R³ have the same meaning as in the above disclosed embodiments, and m is 1. In an embodiment, m is 1 and n is 2. In a particular embodiment, m is 1; n is selected from 0 and 2, R¹ is a hydrogen atom; R² is selected from the group consisting of 5-membered-ring O- or S-heterocycle and a phenyl group, optionally substituted by a (C₁-C₃)-alkoxy group, preferably a methoxy group; and R³ is a phenyl group. Preferably, R² is selected from the group consisting of a furan, a thiophene, a phenyl and a methoxyphenyl group. More preferably, R² is selected from the group consisting of a furan and a methoxyphenyl group.

In a particular embodiment, the PKR1 agonist has the formula (I), (II), (III) or (IV), wherein m, R¹, R² and R³ have the same meaning as in the above disclosed embodiments, and n is 2, with the proviso that when m is 0, R¹ is a hydrogen atom, R² is a furan group and R³ is a phenyl group then the agonist has the formula (III) or (IV). In an embodiment, R¹ is a hydrogen atom; R² is selected from the group consisting of a 5-membered-ring O-or S-heterocycle and a phenyl group, optionally substituted by a (C₁-C₃)-alkoxy group, preferably a methoxy group; and R³ is a phenyl group. Preferably, R² is selected from the group consisting of a furan, a thiophene, a phenyl and a methoxyphenyl group. More preferably, R² is selected from the group consisting of a furan and a methoxyphenyl group.

In a particular embodiment, the PKR1 agonist has the formula (I), (II), (III) or (IV), wherein m, n, R¹ and R² have the same meaning as in the above disclosed embodiments, and R³ is a (C₁-C₆)-alkyl group, optionally substituted by at least one substituent selected from a hydroxyl, a carboxylic group, an aldehyde, a halogen atom and a thiol. In an embodiment, R³ is a (C₁-C₆)-alkyl group. In a particular embodiment, R³ is a tert-butyl group. In a more particular embodiment, R¹ is a hydrogen atom; R² is selected from the group consisting of a 5-membered-ring O- or S-heterocycle, preferably a furan group, and a phenyl group, optionally substituted by a (C₁-C₃)-alkoxy group, preferably a methoxy group; and R³ is a (C₁-C₆)-alkyl group, optionally substituted by at least one substituent selected from a hydroxyl, a carboxylic group, an aldehyde, a halogen atom and a thiol. Preferably, R³ is a (C₁-C₆)-alkyl group. More preferably, R³ is a tert-butyl group.

In a particular embodiment, the PKR1 agonist has the formula (I), (II), (III) or (IV), wherein m, n, R¹ and R² have the same meaning as in the above disclosed embodiments, and R³ is selected from the group consisting of a phenyl group and a 5-membered-ring heterocycle, substituted by at least one substituent selected from the group consisting of a (C₁-C₃)-alkyl, a (C₁-C₃)-alkoxy group and a halogen atom, with the proviso that when m is 0, n is 1, R¹ is a hydrogen atom, R² is a furan group and R³ is a phenyl group substituted by a methoxy, then the methoxy group is in meta- or ortho-position. In an embodiment, R³ is selected from the group consisting of a phenyl group and a phenyl group substituted by at least one substituent selected from the group consisting of a (C₁-C₃)-alkyl, a (C₁-C₃)-alkoxy group and a halogen atom, preferably from the group consisting of methyl, methoxy and chlorine.

In a particular embodiment, the PKR1 agonist has the formula (I), (II), (III) or (IV), wherein m, n, R¹ and R³ have the same meaning as in the above disclosed embodiments, and R² is selected from the group consisting of a 5 to 7-membered-ring S-heterocycle and a phenyl group, optionally substituted by at least one substituent selected from the group consisting of a (C₁-C₃)-alkyl, a (C₁-C₃)-alkoxy group or a halogen atom. In an embodiment, R² is selected from the group consisting of a 5-membered-ring S-heterocycle, preferably a thiophene group, a phenyl and a methoxyphenyl group. In a particular embodiment, m is 1; n is 2; R¹ is a hydrogen atom; R² is selected from the group consisting of a 5-membered-ring S-heterocycle, preferably a thiophene group, a phenyl and a methoxyphenyl group; and R³ is a phenyl group.

The pharmaceutical composition comprising the PKR1 agonist is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art. Possible pharmaceutical compositions include those suitable for oral, rectal, topical (including transdermal, buccal and sublingual), or parenteral (including subcutaneous, intramuscular, intravenous and intradermal) administration. For these formulations, conventional excipient can be used according to techniques well known by those skilled in the art. The compositions for parenteral administration are generally physiologically compatible sterile solutions or suspensions which can optionally be prepared immediately before use from solid or lyophilized form. Adjuvants such as a local anesthetic, preservative and buffering agents can be dissolved in the vehicle and a surfactant or wetting agent can be included in the composition to facilitate uniform distribution of the active ingredient. For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. For transdermal administration, the composition can be formulated into ointment, cream or gel form and appropriate penetrants or detergents could be used to facilitate permeation, such as dimethyl sulfoxide, dimethyl acetamide and dimethylformamide. For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. In a preferred embodiment, the pharmaceutical composition is suitable for parenteral or oral administration.

The PKR1 agonist may be used alone, in combination with one or several other PKR1 agonists and in combination with other active substances, such as drugs used to treat cardiac diseases and/or renal diseases.

The present invention also concerns the use of a PKR1 agonist for preparing a medicament for promoting the differentiation of cardial epicardin+ progenitor cells into cardiomyocytes in a subject affected with a cardiac disease and/or the differentiation of renal epicardin+ progenitor cells into vasculogenic and/or glomerular cells in a subject affected with a renal disease. The PKR1 agonist may be any PKR1 agonist as described above.

The present invention also concerns a method for promoting the differentiation of cardial epicardin+ progenitor cells into cardiomyocytes in a subject affected with a cardiac disease and/or the differentiation of renal epicardin+ progenitor cells into vasculogenic and/or glomerular cells in a subject affected with a renal disease, comprising administering a therapeutically active amount of a PKR1 agonist to said subject. By a "therapeutically effective amount" is intended an amount of PKR1 agonist administered to a patient that is sufficient to provide a therapeutic effect, i.e that is sufficient to promote the differentiation of epicardin+ progenitor cells into cardiomyocytes and/or renal cells. In a particular embodiment, the therapeutically effective amount is 0.2 to 30 mg / kg of body weight / day. It is to be understood that the total amount of the PKR1 agonist may vary depending on the volume of blood plasma of the patient. Suitable means and measures to determine the therapeutically effective amount are available to the person skilled in the art. The PKR1 agonist may be any PKR1 agonist as described above.

The present invention also concerns a PKR1 agonist for use for stimulating the regeneration of cardiomyocytes in a subject having an ischemic heart disease. The regeneration of cardiomyocytes is obtained from the differentiation of cardial epicardin+ progenitor cells. In an ischemic heart disease, and in particular in cardiac infarction, the lack of oxygen caused by obstruction of the tissue's blood supply induces necrosis of cardiomyocytes leading to irreversible injuries. The stimulation of the regeneration of cardiomyocytes allows limiting or preventing these injuries and thus limiting infarction sequelae. The PKR1 agonist may be any PKR1 agonist as described above.

The present invention further concerns a PKR1 agonist for promoting renal angiogenesis, in particular intra- and extra-glomerular angiogenesis. The renal angiogenesis is obtained from the differentiation of renal epicardin+ progenitor cells into renal vasculogenic cells. Promoting renal angiogenesis is particularly important in ischemic renal diseases such as renal artery stenosis which induces intrarenal microvascular and tissue remodeling that may lead to irreversible injury and progressive deterioration of renal function. The PKR1 agonist may be any PKR1 agonist as described above.

The present invention further concerns a PKR1 agonist for use for the prevention or the treatment of a renal disease. As used herein, the term "prevention or treatment of a disease" or "to prevent or to treat a disease refers to any act intended to ameliorate the health status of patients such as therapy, prevention, prophylaxis and retardation of the disease or the disorder. In certain embodiments, such term refers to the amelioration or eradication of the disease or the disorder, or symptoms associated with said disease or disorder. In other embodiments, this term refers to minimizing the spread or worsening of the disease or disorder resulting from the administration of one or more therapeutic agents to a subject with said disease or disorder. The renal disease is as defined above. The PKR1 agonist may be any PKR1 agonist as described above.

The inventors have herein demonstrated that PKR1 agonists promote the differentiation of cardiac epicardin positive progenitor cells (cEPPC) into cardiomyocytes and, in the same time, block the differentiation of these cells into adipocytes. Accordingly, the present invention concerns a PKR1 agonist for use for preventing fat tissue development in heart and/or kidney in a subject, in particular in a subject affected with obesity or being susceptible to develop obesity. Obesity is a disease due to excess body fat leading to reduced life expectancy and increased health problems. As used herein, the term "subject affected with obesity" or "obese subject" refers to a subject having a Body Mass Index (BMI) that is greater than 30 kg/m², preferably 35 kg/m², more preferably 40 kg/m². The PKR1 agonist may be any PKR1 agonist as described above.

The present invention also concerns a PKR1 agonist for use for preventing epicardial fat tissue development in heart in a subject affected with or being susceptible to a cardiac disease. In a particular embodiment, the subject affected with or being susceptible to a cardiac disease is an obese subject. The present invention also concerns a PKR1 agonist for use for preventing fat tissue development in kidney in a subject affected with or being susceptible to a renal disease. In a particular embodiment, the subject affected with or being susceptible to a renal disease is an obese subject.

In another aspect, the present invention further concerns an *in vitro* or *ex vivo* method of producing cardiomyocytes, wherein said method comprises the step of contacting cardial epicardin+ progenitor cells with a PKR1 agonist. The present invention also concerns an *in vitro* or *ex vivo* method of producing glomerular cells, wherein said method comprises the step of contacting renal epicardin+ progenitor cells with a PKR1 agonist.

The culture medium which may be used during the step of contacting epicardin+ progenitor cells with a PKR1 agonist is designed to support the growth and the differentiation of these cells. This medium generally is changed every day and comprises a carbon source, a nitrogen source, antibiotics to prevent fungi and bacteria growth, a buffer to maintain pH and specific growth factors. This medium may be easily designed by the skilled person in the art. In an embodiment, the culture medium comprises compounds for stimulating cell differentiation selected from the group consisting of dexamethasone, ascorbic acid (vitamin C) and glycerophosphate, and combinations thereof. In a preferred embodiment, the culture medium comprises dexamethasone, ascorbic acid and glycerophosphate. The PKR1 agonist may be any PKR1 agonist as described above.

A pharmaceutical composition comprising cardiomyocytes and/or glomerular cells obtained with the method of the invention may be administered to a subject in need thereof, i.e. in a subject affected with a cardiac and/or renal disease. Cells may be administered with a pharmacologically acceptable carrier that is compatible with the cells and may be, for instance, cell culture medium (such as Eagle's minimal essential media), phosphate buffered saline, Krebs-Ringer buffer, and Hank's balanced salt solution with glucose (HBSS). The pharmaceutical composition comprising cells is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art. In a preferred embodiment, pharmaceutical composition is suitable for parenteral administration. In a particular embodiment, the pharmaceutical composition comprises cells obtained by the method of the invention encapsulated in a biocompatible matrix known in the art. A variety of encapsulation technologies have been developed (e.g. Qi et al., 2008 and WO 91/10425).

The present invention also concerns a method of treating cardiac disease in a subject in need thereof, said method comprising steps consisting of
- obtaining cardiac epicardin+ progenitor cells;
- contacting said epicardin+ progenitor cells with a PKR1 agonist for promoting the differentiation of said cells into cardiomyocytes;
- transplanting a therapeutically effective amount of cardiomyocytes obtained by differentiation of said epicardin+ progenitor cells into said subject.

The present invention further concerns a method of treating renal disease in a subject in need thereof, said method comprising steps consisting of
- obtaining renal epicardin+ progenitor cells;
- contacting said epicardin+ progenitor cells with a PKR1 agonist for promoting the differentiation of said cells into glomerular cells;
- transplanting a therapeutically effective amount of glomerular cells obtained by differentiation of said epicardin+ progenitor cells into said subject.

The PKR1 agonist may be any PKR1 agonist as described above.

In a preferred embodiment, cardiomyocytes or glomerular cells are administered by injection. Preferably, these cells are injected directed into the heart or into the kidney.

According to the origin of epicardin+ progenitor cells, the transplantation may be autologous, isogeneic, allogeneic or xenogeneic. As used below, the "donor" is the donor of epicardin+ progenitor cells and the "recipient" is the subject who receives the transplantation. The transplantation may be isogeneic, i.e. the donor and recipient are genetically identical; allogeneic, i.e. the donor and recipient are of the same species; xenogeneic, i.e. the donor and recipient are of different species; or autologous, i.e. the donor and recipient are the same subject. Allogeneic and xenogeneic transplantations may require the administration of antirejection drugs. For isogeneic, allogeneic and xenogeneic transplantations, the donor may be alive or deceased. In a preferred embodiment, the transplantation is autologous.

In another aspect, the present invention concerns a PKR1 agonist for use for preventing or limiting the nephrotoxicity of a compound, in particular of a drug. The present invention also concerns the use of a PKR1 agonist for preparing a medicament for preventing or limiting the nephrotoxicity of a compound, in particular of a drug. The present invention further concerns a method for preventing or limiting the nephrotoxicity of a compound in a subject, in particular the nephrotoxicity of a drug, comprising administering a therapeutically active amount of a PKR1 agonist to said subject. In this aspect, by a "therapeutically effective amount" is intended an amount of PKR1 agonist administered to a subject that is sufficient to limit or prevent an alteration of the renal function. For example, the nephrotoxic compound may be selected from the group consisting of aminoglycoside antibiotics such as amikacin, arbekacin, gentamicin, kanamycin, neomycin, netilmicin, paromomycin, rhodostreptomycin, streptomycin, tobramycin, and apramycin; diuretics; angiotensin-converting enzyme inhibitors such as Captopril; cyclosporin A; FK-506; Amphotericin B; Cisplatin; Nonsteroidal antiinflammatory drugs such as aspirin, ibuprofen; antibacterial sulfonamides; and antineoplastic agents, in particular anthracyclines. The PKR1 agonist may be any PKR1 agonist as described above.

The following examples are given for purposes of illustration and not by way of limitation.

### Examples

### Example 1: Effects of PKR1 agonists on cardiac epicardin positive progenitor cells

### Materials and Methods

### Extraction and culture of cEPPC

Hearts were removed from P1 neonate C57BL/B6 mice or PKR1 null mutant mice, cut into pieces (approximately 1 mm³), rinsed in PBS to remove excess blood and plated onto 0.1 % gelatin petri dish in DMEM containing GlutaMax, 4.5 g /L glucose, penicillin, streptomycin and 15 % fetal calf serum. Cultures were maintained with minimum disturbance to allow explants to adhere. All cells were maintained at 37°C in a humidified air 5% CO₂ atmosphere. After 4 days explants were removed and cells were gently washed and fresh medium with DMEM containing GlutaMax, 4.5 g /L glucose, penicillin, streptomycin and 10 % fetal calf serum serum was added. The cells were cultured during 10 days and trypsinized for experimentation.

### Adipogenic treatment

For adipogenesis, cells were plated at the density of 2x10⁴ onto 0,1% gelatin coated culture chamber in DMEM containing GlutaMax, 4.5 g /L glucose, penicillin, streptomycin and 10 % fetal calf serum. After 4 days the fresh medium was added and adipocyte differentiation wad induced on confluent cells by treatment with adipogenic cocktail comprising 0.25µM Dexamethasone, 0.5mM 3-isobutyl-I-methylxanthine, 10 µg/ml insulin during 48 hours at 37°C in a humidified air 5% CO₂ atmosphere. After 48 hours the fresh medium was added and the adipogenesis was maintained by adding 10µg/ml insulin during 48 hours. After this time the medium was changed and the adipogenesis was continued during 48 hours. The induction of adipogenesis was repeated by a new treatment with 0.25 µM Dexamethasone, 0.5mM 3-isobutyl-I-methylxanthine, 10µg/ml insulin during 48 hours following by 10µg/ml insulin treatment during 48 hours and finally the cells were maintained in fresh medium for 48 hours. To test the role of Prokineticin-2 in adipogenic differentiation of EPPC, the cells were treated with prokineticin-2, 12 hours before the adipogenesis beginning (including the treatment by 0.25 µM Dexamethasone, 0.5 mM 3-isobutyl-1-methylxanthine, 10 µg/ml insulin) for the two steps.

### Oil red O straining

Cells were fixed in 3.7 % formaldehyde at 4°C during 1 hour. The accumulation of neutral lipids can be detected by staining the cells in solution of 0.5% Oil red O in 60% isopropanol at room temperature during 30 minutes. To quantify the cells differentiation DAPI (a marker of nucleus) was added at 1/100 in water. The pictures were taken with inverted microscope at x40 objectif.

### Rna Isolation

After adipogenic treatment the total RNA from cells was isolated using TRI Reagent (Molecular Research Center) and treated with DNase using the RNase-Free DNase Set. The quantitative PCR reactions were carried out in the presence of a fluorescent dye (Sybrgreen, BioRad). Relative abundance of mRNA was calculated after normalisation to 36B4. The expression of PPAR alpha and PPAR gamma was analyzed.

| | |
|---|---|
| Mouse 36B4 forward | 5'AGA TTC GGG ATA TGC TGT TGG 3' (SEQ ID NO. :1) |
| Mouse 36B4 reverse | 5'AAA GCC TGG AAG AAG GAG GTC 3' (SEQ ID NO. :2) |
| Mouse PPAR alpha forward | 5'ACG ATG CTG TCC TCC TTG ATG 3' (SEQ ID NO. :3) |
| Mouse PPAR alpha reverse | 5'GTG TGA TAA AGC CAT TGC CGT 3' ((SEQ ID NO. :4) |
| Mouse PPAR gamma forward | 5'CCA TTC TGG CCC ACC AAC 3' (SEQ ID NO. :5) |
| Mouse PPAR gamma reverse | 5'AAT GCG AGT GGT CTT CCA TCA 3' (SEQ ID NO. :6) |

### Cardiomyocyte treatment

For cardiomyogenesis, cells were plated at 2x10⁴ onto 1% gelatin coated culture chamber in DMEM containing GlutaMax, 4.5 g /L glucose, penicillin, streptomycin and 10 % fetal calf serum. The cells were maintained in culture during six days and medium were change every 2 days. After 6 days, cardiomyocyte differentiation can be induced using culture medium supplemented with 1µM dexamethasone, 50µg/ml ascorbic acid, 10mM glycerophosphate without or with 10 nM of prokineticin-2. The medium was change every 2 days during 36 days. After this time, the contractility of cardiomyocyte was taken with time laps microscopy at 5% CO₂ 37°C, and 800 frames take per cardiomyocyte group (10ms/frame). To test the contractility of cardiomyocyte (beating cells), a beta adrenergic receptor agonist was applied into the medium at the concentration of 25 nM for Isoproterenol and then time laps analyses were performed.

### Immunostaining

Cells were fixed in 3,7% formaldehyde at 4°C 1 hours. After fixation, the cells were blocked with in PBS containing 10% donkey serum and 0.5 % Triton-X100 for 1 hour and then incubated with antibody against Troponin C (Santa Cruz), containing 1% donkey serum and 0.05% Triton-X100 at 4°C overnight. The following secondary antibodies were used Alexa 488-congugated anti goat antibody. Finally, cells were covered with mounting medium from Vector Laboratories containing 4, 6-diamidio- 2- phenylindol dihydrochliride (DAPI). The immunofluorescence was detected by Leica TCSNT fluorescent microscope.

### Results

### Cardiac epicardin positive progenitor cell (cEPPC) differentiation into adipocytes

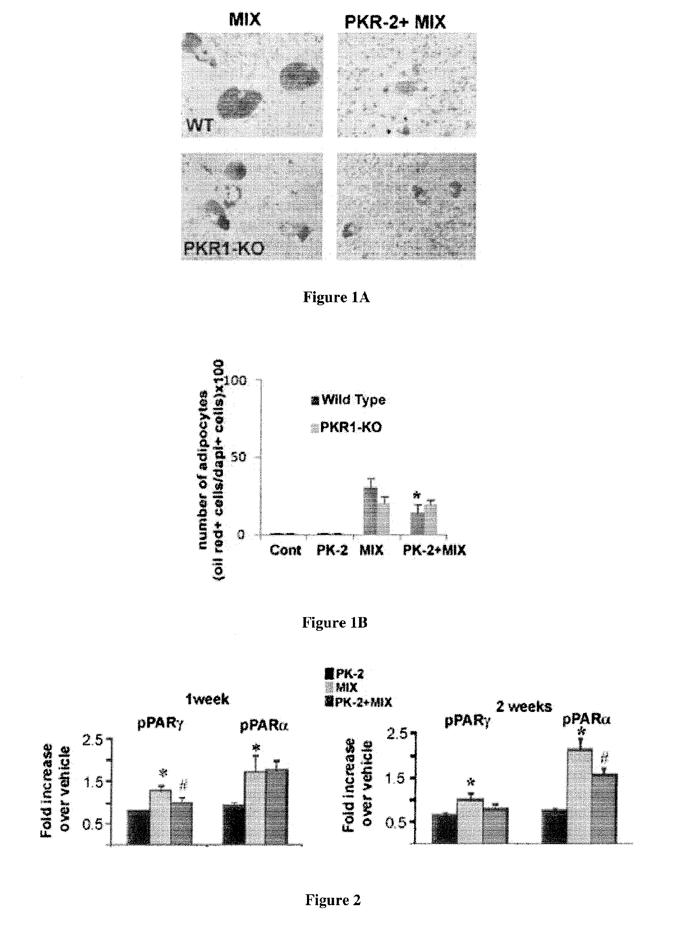

Neonatal EPPC were subjected to the standard differentiation program including dexamethasone, 3-isobutyl-1-methylxanthine, and insulin. For the first time, it was shown that the stimulation of cEPPC by proadipogenic treatment leads to adipocyte formation (Fig. 1A, upper line, left panel; Fig. 1B).

It was also determined whether prokineticin-2 alters cEPPC differentiation into adipocyte. To explore this phenomenon, cEPPC were pre-treated with prokineticin-2 several hours before pro-adipogenic treatment. It was thus demonstrated that prokineticin-2 pre-treatment inhibits adipocyte formation in cEPPC (Fig. 1A, upper line, right panel; Fig. 1B).

### Involvement of PKR1 in inhibition of adipogenesis

The role of PKR1 in inhibition of adipogenesis by prokineticin-2 was tested by using the same differentiation protocol for cEPPC derived from PKR1 null mutant mice (cEPPC-PKR1-/-). It was thus shown that there was no difference in adipocyte formation in cEPPC-PKR1-/- without or with prokineticin-2 pretreatment during adipogenic stimuli. These data demonstrated that prokineticin-2 acts via PKR1 to inhibit the adipocyte formation in cEPPC (Fig. 1A, lower line; Fig. 1B).

### Regulation of adipogenic gene expression

Since the program of adipocyte differentiation involves up-regulation of a subset of gene such as pPAR gamma and pPAR alpha, that are specific for maturing or mature adipocyte, it was tested whether the expression of mRNAs of these genes correlated with the inhibition of differentiation. The inventors showed that a pro-adipogenic treatment in cEPPC was correlated with up-regulation of adipogenic genes such as PPAR gamma in early stage of adipogenesis following by PPAR alpha in late stage of adipogenesis. Interestingly, they also showed that prokineticin-2 pretreatment leading to adipocyte regression involved in suppression of PPAR gamma gene expression in early stage of adipogenesis. They revealed in the same condition, that prokineticin-2 pretreatment inhibits up-regulation of PPAR alpha in the late stage of cEPPC adipogenesis (Figure 2).

### Cardiomyocyte differentiation

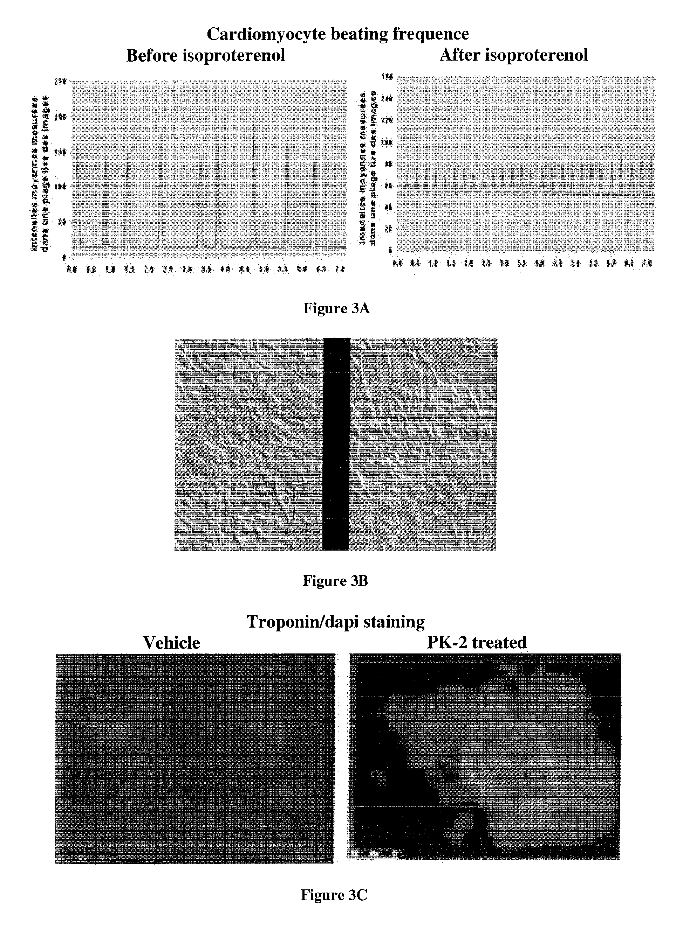

The ability of cEPPC to differentiate into cardiomyocyte lineage was testes. The inventors showed that a treatment comprising Dexamethasone, acid ascorbic and glycerophosphat alone can not lead to differentiation of cEPPC into functional cardiomyocytes. Surprisingly, they demonstrated that a treatment of prokineticin-2 leads to differentiation of cEPPC into functional cardiomyocytes (Figure 3).

### Conclusion

The inventors have herein demonstrated that PKR1 agonists stimulate the differentiation of cardiac EPPC in cardiomyocytes and block their differentiation in adipocytes.

### Example 2: Effect of genetic inactivation of PKR1 and activation of PKR1 signaling Pathway in kidney

### Materials and Methods

### Generation of knockout mice

To conditionally disrupt the GPR73 gene encoding PKR1, embryonic stem (ES) cells were electroporated with the targeting vector pGPR73 L3 that encompasses GPR73 exon 2 and contains a loxP site in the intron located upstream of exon 2, whereas a loxP-flanked (floxed) neomycin (neo) selection cassette is present in the intron downstream of exon 2. To excise floxed DNA segments of the GPR73 L3 allele, GPR73 L3/+ mice were bred with CMV-Cre transgenic mice that express Cre recombinase in germ cells. PCR analysis of tail DNA revealed a Cre-dependent GPR73 ^{L-} allele excision, yielding GPR73^{+/L-}heterozygous animals. After heterozygous interbreeding, GPR73^{L-/L-} mice were recovered at Mendelian frequency, survived to adulthood, and were fertile. These mice have a reduced life span by 6% as compared to wild type.

### TUNEL Analysis

Briefly, frozen sections were fixed in 4% freshly prepared paraformaldehyde in PBS for 20 minutes and incubated in permeabilization solution (0.1 % Triton, 0.1 % sodium citrate), followed by incubation with TUNEL reaction mixture at 37°C for 60 minutes in a dark, humidified chamber. Sections were then washed, counterstained with DAPI (0.5 µg/mL in PBS), and mounted with VECTASHIELD medium (Vector Laboratories, Inc, Burlingame, Calif).

For staining the tissue section for succinate dehydrogenase activity, the kidneys were frozen, and 5-µm sections were incubated in 0.1 M phosphate buffer (pH 7.4) containing 0.1 M sodium succinate and 0.1% nitro blue tetrazolium at 37 °C for 5 min. The staining intensity (blue color) of each section was quantified by using Image software. 6 to 10 images per kidney (3 to 4 animals per genotype group) were acquired with a fluorescence microscope (Leica). Results are expressed as the percentage of apoptotic cells. The TUNEL labeling index was calculated as the mean number of DAPI-stained TUNEL-positive nuclei/glomerulus, for 50 glomeruli per kidney (n=3) for the various groups of mice.

Glomerular pathology analyses were based on the assessment of 50 glomeruli/kidney cross-section. Tubule damage (consisting of at least one of the following: dilatation, atrophy, necrosis) was assessed by scoring 100 renal proximal tubules/kidney cross-section on randomly selected high-power microscopy fields (x40).

Frozen tissue sections for the immunofluorescence staining of structural proteins were fixed, blocked and stained with primary antibodies against PECAM-1, podocin, NaPi, Wt1, Dystrophin, N-cadherine and PKR2, (Santa Cruz); LC3, and epicardin (abcam), α-SMA (sigma), active capase-3 (Millipore), and PKR1 (IGBMC, Illkirch). Antibody binding was detected by incubation with Fluorescein, Alexa 555- or Alexa 488-conjugated secondary antibodies. Finally, the nuclei were stained with DAPI. Fluorescence was analyzed on a Leica Microsystems TCS SP5 laser scanning confocal microscope. The number of PECAM-1-positive, DAPI-stained cells per glomerulus, was determined by counting 50 glomerular fields per kidney section for each group of mice.

For staining the tissue section for oil red O, cryosectiones (5 µm) were fixed in 10% formalin for 30 min, and then washed in distilled water and stained with 0.5% oil red O in 36% Triethylphosphat (Sigma-Aldrich) for 30 min to identify neutral lipids, cholesterol, and fatty acids (red colour). After rinsing with water, nuclei were counterstained (blue) with Mayer's hematoxylin for 5 min and washed in distilled water for 3 times. Finally, slides were covered in aqueousmount under a coverslip for viewing with a light microscope at equal light intensity. Images from the stained slides of mice were initially acquired using a 24-bit file format. ImageJ 1.37v software (National Institutes of Health) was used to convert bright-field (24-bit) images of Oil red O stainings to 8-bit images. Threshold values were chosen that maximize selection of the Oil red O positive tissue while minimizing background interference. Thus, the total number of lipid drops from each image (percent) was quantified.

### RNA Extraction, Quantification, and Reverse-Transcription Polymerase Chain Reaction Analysis

A minimum of 3 mouse kidney samples for each of the genotype groups were analyzed in each experiment. Extracted RNA samples were used as the template for cDNA prepared via a reverse-transcription polymerase chain reaction that was performed in duplicate for all RNA samples. Semi-quantitative or real-time reverse-transcription polymerase chain reaction (MiQ, Biorad) was then used to determine the mRNA expression of indicated genes. The GAPDH housekeeping gene was used as a control. Results were analyzed with Bio-Rad qPCR software version 2.0.

### In situ hybridization

DNA templates for producing mouse PKR1 riboprobes were generated from PCR expression vector encoding full length PKR1 after the linearization with BamHI or EcoRV and transcription with T7 or Sp6 RNA polymerases to generate the antisense and sense probes, respectively. Digoxigenin-labeled (DIG-RNA labeling Mix, Roche) riboprobes were transcribed in vitro in antisense or sense orientations using T7, Sp6, or T3 RNA polymerases as indicated above. Some of the experiments were performed using a Tecan GenePaint Robot and a Tyramide signal amplification method (for details, see www.eurexpress.org and www.genepaint.org). In each hybridization experiment, control sense probes were included, allowing the determination of possible false-positive signal.

### Western blot analysis

Tissues were lysed in lysis buffer. Total protein was separated by SDS-PAGE in 7 or 12% acrylamide gels and blotted onto PVDF membranes. The membrane was blocked and incubated with primary antibodies, troponin C, HIF1 α (Santa Cruz); phosho-Akt and total Akt (Cell Signaling). It was then incubated with horseradish peroxidase-conjugated anti-rabbit or anti-mouse secondary immunoglobulins. The signal was then detected by enhanced chemiluminescence (Pierce, Rockford, IL), according to the manufacturer's protocol. Akt activity was evaluated and quantified by Western blot analysis and densitometry, using antibodies specific for Akt kinase at the residues (Ser-473) that are phosphorylated upon activation.

### Biochemical analyses on blood and urine samples

After an overnight fast, blood was collected at 8 a.m. by retro-orbital puncture after a short anesthesia with isoflurane (Forene®, Abbott France). 300 µl of blood was collected into a heparinized tube for biochemical analysis. Urine was obtained from individual mice housed in metabolic cages for 24 hours. Urine and plasma creatinine concentrations were determined by a modification of Jaffé's reaction method. The parameters were determined using an Olympus analyzer with kits and controls supplied by Olympus or other suppliers (see EMPReSS Website).

### Kidney explants, Isolation of Epicardin + progenitor cells

Kidneys were removed from P1 neonate C57BL/B6 mice, cut into pieces (approximately 1 mm³), rinsed in PBS to remove excess blood and plated onto 0.1 % gelatin-coated culture chamber in DMEM containing GlutaMax^{™}, 4.5 g /L glucose, penicillin, streptomycin and 15 % fetal calf serum (FCS) in the presence of prokineticin-2 (5 nM-10 nM) or its vehicle (PBS only). Cultures were maintained with minimum disturbance to allow explants to adhere. After 2 or 4 days of culture, explants were removed and cells were maintained in DMEM containing Glutamax, 4.5g/L glucose, penicillin streptomycin without serum during 12hours at 37°C in a humidified air 5% CO₂ atmosphere. Next, cells were cultured in the presence or absence of prokineticin-2 (5 or 10nM) during 24 hours. Cells were fixed in 3.7% formaldehyde at 4°C and cell types were assessed by immunofluorescence.

Primary renal-EPDC cells migrated away to form a monolayer surrounding the remaining kidney explants were utilized and further cultured (16 days) in DMEM containing GlutaMax^{™}, 4.5 g /L glucose, penicillin, streptomycin and 10% FCS, and trypsinized and resuspend in the isolation Buffer (0.1%BSA, 2mM EDTA in PBS) and incubated with biotynilated anti-TCF21 for 20 minutes at room temperature then incubated with 85µl of FlowComp Dynabeads (Dynabeads R FlowCompTM Flexi, invitrogen) for 30 minutes at room temperature and then placed in the magnet for 2 minutes. The cells attached to Dynabeads were collected with 1mL FlowComp Release Buffer and then cells were resuspend in medium containing 10% Fetal calf serum with DMEM 4.5g/L glucose, glutamax, pencilline, streptomycin. After 12 hours cells were washed with PBS and fresh medium without serum were added during 12 hours. Next, cells were cultured in the presence or absence of prokineticin-2 (5 or 10nM) during 48 hours. Cells were fixed in 3.7% formaldehyde at 4°C and cell types were assessed by immunofluorescence staining.

### Statistical analysis

Data are expressed as means ± SEM. Multigroup comparisons were carried out by one-way ANOVA with post hoc correction. Student's t test was used for pairwise comparisons between groups. For all analyses, values of P<0.05 were considered significant.

### Results

### PKR1 deficiency-induced renal abnormalities accompanied apoptosis and reduced capillary lumen in glomeruli

Macroscopic inspection showed the kidneys of PKR1 null mutant mice to be massively enlarged, with an irregular surface presenting multiple cortical swellings (Figure 4A). In some cases, one kidney was atrophied, or displayed massive urine retention (Figure 4A and B). Overall, kidney weight with urine in the cortical swellings to body weight ratio significantly increased in PKR1-null mice as compared to wild type mice at the age of 24 weeks (Figure 4C). The inventors also examined neonatal kidneys, to determine the clinical course of the disease. Kidney hypoplasia was also evident in neonatal mutants (Figure 4D). TUNEL assays in kidney sections from mutant and wild-type mice revealed that the glomeruli of mutant kidneys contained significantly more TUNEL-positive cells than those of wild-type kidneys, at the age of 24 weeks (Figure 4E). Accordingly, mutant glomeruli contained fewer PECAM-1-positive visible capillary endothelial cells (Figure 4F).

### Renal ultrastructural analysis and abnormal mitochondrial

Systematic histological examinations of PKR1 null mutant mice showed dilatation of the Bowman's spaces in glomeruli (Figure. 5A, upper panels). Electron micrographs showed considerable thickening of the lamina densa of the glomerular base membrane (GBM), with the presence of numerous protusions on the podocytic side (Figure 5A, lower panels). Mallory tetrachrome staining revealed dilated renal tubules and severe interstitial fibrosis between the tubules (Figure 5B, upper panels). In the mutant kidney, peritubular capillaries were occupied by structures with multiple lumens and prominent interstitial cells, often in loose associations, corresponding to dysmorphogenic capillaries (Figure 5B, middle panels). The number and density of mitochondria in proximal tubule cells was significantly lower in mutant than in wild-type animals. These abnormalities in mitochondrial density were accompanied by changes in mitochondrial structure, such as mitochondrial swelling, and decreases in the numbers of cristae and granulocytes (Figure 5C, lower panels). Consistent with the observed defects in mitochondrial morphology, mutant kidneys displayed only 75% of the SDH activity as compared to wild-type kidneys (n=6, Figure 5D).

### PKR1 deficiency-induced renal dysfunction

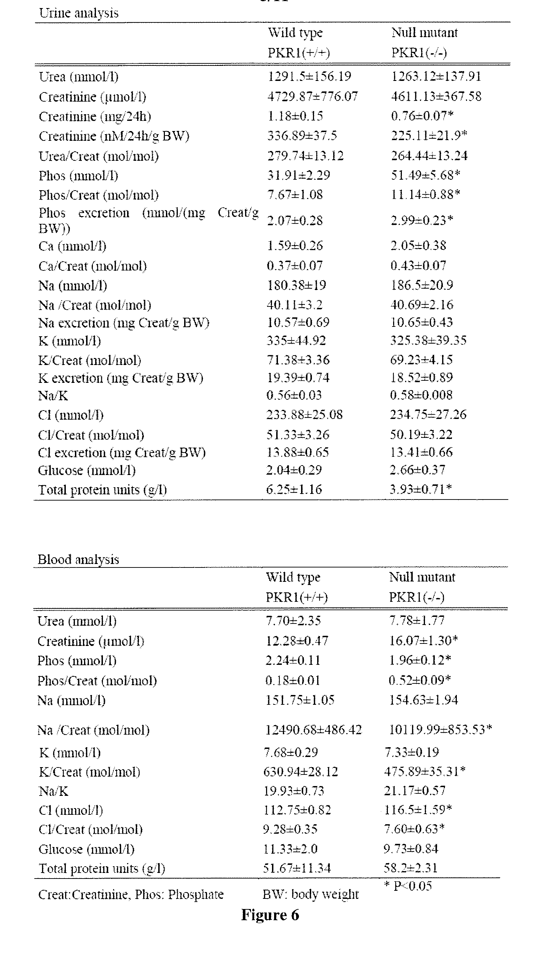

Renal functions of 24 weeks old mice were assessed by blood and urine chemistry. The 24-h urine flow of PKR1 null mutant mice was significantly lower than that of wild-type mice. However, no differences in water and food intake and blood glucose levels were observed (Figure 6). No significant changes in urinary potassium, sodium and chloride excretion rates were observed between mutant and wild-type mice, consistent with the values obtained for serum (Figure 6). Mutant mice had a significantly lower glomerular filtration rate, as estimated by creatinine clearance. Lower creatinine concentrations (mmol/24-h/g) in the urine of mutant mice were associated with higher concentrations in the serum of these mice. However, the urine phosphate/creatinine, but not calcium/creatinine ratio was significantly higher in mutant mice than in wild-type controls. Mutant mice displayed higher levels of absolute renal phosphate (Pi) excretion, associated with hypophosphatemia (Figure 6), indicating that mutant mice displayed poor Pi retention in the kidney and poor stabilization of serum Pi concentration. Mutant mice exhibit protein urea at the age of 36 weeks old (total proteins in urine were 6 625±0.7 and 10.72±2.4g/l, for wild type and mutant, respectively, n=5, p<0.05), consistent with fenestrated endothelial cell structures at this age (Figure 7).

### Characterization of cardiac and renal function impairment

The potential defect in vascularization was determined by determining numbers of PECAM-1+capillary density in kidneys. Neonatal PKR1-null mutant mice exhibit reduced numbers of capillary number ad density as detected by PECAM-1 staining in kidneys as compared to their wild-type littermates (Figure 8A). A reduction of angiogenesis was observed in mutant glomerulus by 39% (n=4, p<0.05). Hypoxia inducible factor (HIF-1 α) transcript levels did not significantly increase in the mutant kidneys at the postnatal day 1. However, at the age of three weeks, inductions of HIF-1 α transcript as well as protein were observed in kidneys of mutants (Figure 8B, C). Parallel to HIF-1α induction at this age, pro-angiogenic factors, PDGF-B and FGF-2 transcripts were increased in mutant kidneys. Furthermore, enhancement of VEGF transcripts were significantly in the kidneys of the mutants (Figure 8D). This data indicates the initiation of a compensatory signaling at these time points.

Next, the inventors investigated whether PKR1 survival pathway is impaired in the neonatal kidney. Indeed, activation of Akt kinase appeared reduced in mutant kidneys as compared to wild type, indicated by 60% or greater reductions in phospho-Akt (Figure 8F), confirming the increased cell death in this organ at the early stage. Co-staining of the apoptotic marker, active caspase-3 and cell specific markers illustrated that glomerular progenitor cells (epicardin+) and glomerular endothelial cells (pecam+) were also positive for active caspase-3 (Fig. 8F) in only mutant kidneys at the 24 weeks-old-age. These data clearly indicate that the angiogenic defects in mutant are ameliorated over time but cannot compensate for the survival deficit in these cells. Supporting this findings, the inventors found that VEGF level is increased in extraglomerular area, but not in glomerular zone of the mutant kidneys (Figure 8G).

Interestingly, epicardin+ cells in the mutant glomerulus were significantly lower than those in the wild-type tissues (Figure 9A). To assess the possible direct effect of PKR1 signaling on renal-epicardin+ cells as shown for cardiac-epicardin+ cells (Urayama et al., 2008), the inventors used explant cultures from wild-type and mutant kidneys from postnatal day 1 (P1). In untreated kidney explants, 50±6 % of the cells proliferates, as shown by Ki67 staining (Figure 9B). The treatment of kidney explants with prokineticin-2 (5-10 nM) stimulated the extensive outgrowth of cells (Figure 9B, histogram). 90% of the emerging epithelial cells were positive for epicardin. Prokineticin-2 induced differentiation of renal-epicardin+ cells into PECAM-1+ endothelial and α-SMA+ vascular smooth muscle cells that were blocked in the mutant renal-epicardin+ cells (Figure 9B, histogram). Similar results were obtained in epicardin+ isolated cell cultures derived from kidney explants.

### Conclusion

The inventors have herein provided the first evidence that angiogenic PKR1 signaling regulates heart kidney functions. They further showed that PKR-1 null mice develop also renal functional abnormalities.

The inventors have also herein provided the first evidence that epicardin+ progenitor cells can be found in kidney. The data presented here are also the first *in vivo* report that PKR1 signaling prevents apoptosis in glomerular endothelial and progenitor cells. The inventors have further demonstrated that PKR1 signaling controls epicardin+ progenitor cell differentiation that is involved in glomerular angiogenesis.

### Example 3: Non peptide PKR1 agonists

### Materials and Methods

### Synthesis and NMR characterization of PKR1 agonists

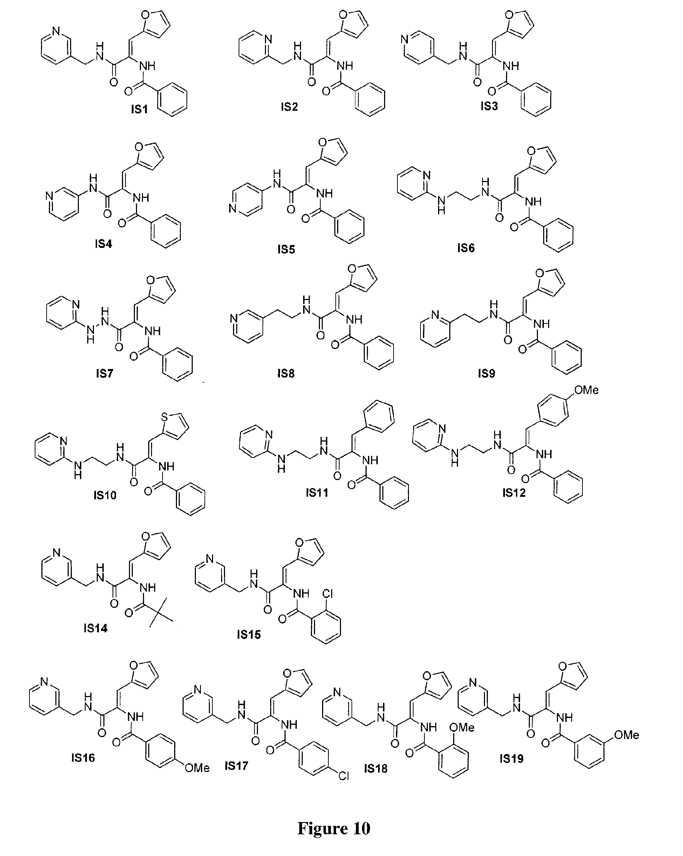

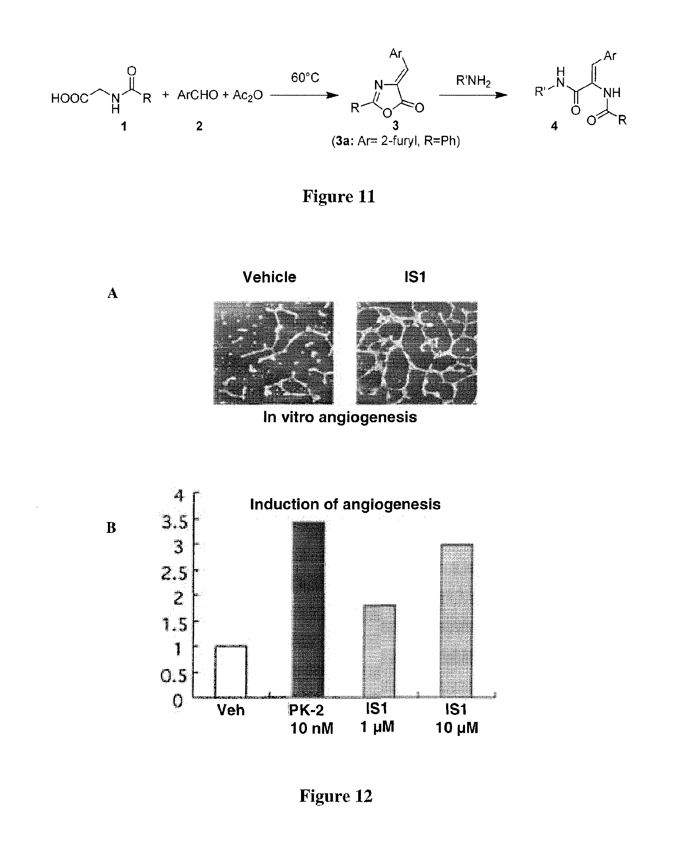

IS compounds (Figure 10) were synthesized according to a general method originally developed by Erlenmeyer and Plöchl (Erlenmeyer, 1893; Plöchl, 1884). Condensation of acylated glycine **1** with an aldehyde **2** in the presence of acetic anhydride afforded the azlactone **3**, which was condensed to an amine to afford **4** (Figure 11).

### ISI to IS3, IS6, IS8 to IS12 and IS14 to IS19

An amine (4 mmol) and an azlactone **3** (4 mmol) in suspension in CH₂Cl₂/EtOH (8 mL, 1/1) was sonicated for 10 min. After stirring overnight at room temperature, the solvent was removed under vacuum, and the residue was purified by flash chromatography (EtOAc to EtOAc/EtOH, 80/20) to give the expected product a white solid (purity > 97%).

| | |
|---|---|
| 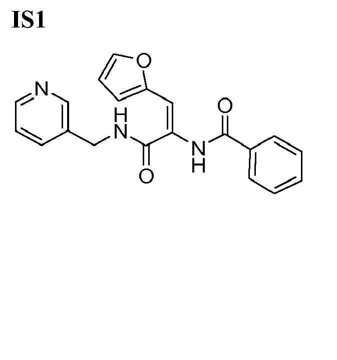 | **¹H NMR** (400 MHz, DMSO-d₆): 9.77 (s, 1H), 8.68 (m, 1H), 8.58 (s, 1H), 8.47 (d, *J* = 4.6 Hz, 1H), 8.13 (m, 2H), 7.77 (d, *J* = 7.8 Hz, 1H), 7.62 (m, 2H), 7.55 (m, 2H), 7.33 (m, 2H), 6.69 (d, *J* = 3.4 Hz, 1H), 6.52 (m, 1H), 4.49 (d, *J* = 6.0 Hz, 2H); **RMN ¹³C** (100 MHz, DMSO): 40.3, 111.7, 113.5, 117.7, 122.8, 126.8, 127.7, 127.8, 131.2, 133.6, 134.7, 143.6, 147.5, 148.5, 149.6, 164.4, 165.7 ppm. LC-MS (ESI): calculated: 347.1; found: 348.0 (M+H⁺)⁺. |
| 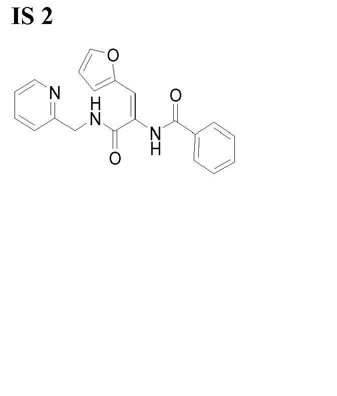 | **¹H NMR** (400 MHz, CDCl₃ ): 8.57 (s, 1H), 8.42 (d, *J* = 4.9 Hz, 1H), 7.92 (d, *J* = 7.5 Hz, 2H), 7.61 (m, 2H), 7.53 (d, *J* = 7.8Hz, 1H), 7.43 (m, 3H), 7.32 (d, *J* = 7.8 Hz, 1H), 7.11 (m, 1H), 6.94 (s, 1H), 6.49 (d, *J* = 3.5, 1H), 6.41 (dd, *J* = 1.9,3.6 Hz, 1H), 4.33 ( d, *J* = 5.3 Hz, 2H); **¹³C NMR** (100 MHz, CDCl₃):166.5, 165.1, 156.8, 150.5, 149.0, 144.1, 137.0, 133.6, 132.4, 128.9, 127.8, 127.4, 122.4, 122.2, 114.6, 114.0, 112.3, 45.1. LC-MS (ESI): calculated: 347.1; found 348.2 (M+H⁺)⁺. |
| 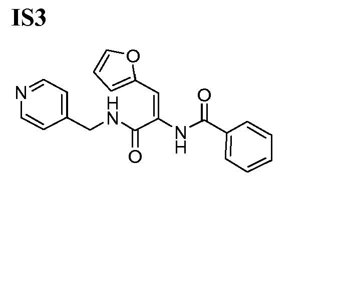 | **¹H NMR** (300 MHz, CDCl₃): 8.59 (s, 1H), 8.49 (m, 2H), 7.93 (m, 2H), 7.56 (m, 1H), 7.47 (m, 3H), 7.25 (s, 1H), 7.12 (m, 1H), 6.94 (s, 1H), 6.52 (d, *J* = 3.4 Hz, 1H), 6.44 (m, 1H), 4.53 (d, *J* = 6.2 Hz, 2H); **¹³C NMR** (100 MHz, DMSO-d₆): 165.9, 164.7, 149.5, 148.9, 148.1, 143.5, 133.3, 131.2, 127.8, 127.5, 126.4, 121.7, 117.3, 113.6, 111.6, 41.7. LC-MS (ESI): calculated 347.1; found: 348.2 (M+H)⁺. |
| 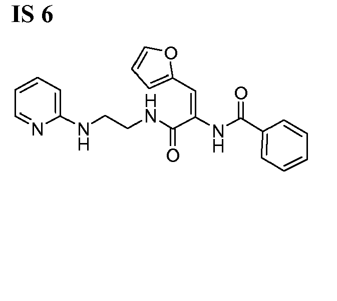 | **¹H NMR** (400 MHz, CDCl₃): 8.75 (s, 1H), 7.87 (m, 3H), 7.58 (m, 1H), 7.48 (t, *J* = 7.4 Hz, 1H), 7.38 (m, 3H), 7.24 (m, 1H), 6.69 (s, 1H), 6.38 (m, 4H), 5.29 (m, 1H), 3.46 (m, 4H); **¹³C NMR** (100 MHz, CDCl₃): 166.4, 165.8, 159.0, 150.4, 147.6, 143.9, 137.3, 133.3, 132.4, 128.8, 127.8, 127.6, 114.2, 113.3, 112.8, 112.2, 108.6, 41.1, 41.0. LC-MS (ESI): calculated: 376.2; found: 377.2 (M+H⁺)⁺. |
| IS 8 | **¹H NMR** (400 MHz, CDCl₃): 8.46 (s, 2H), 8.41 (m, 1H), 7.91 (m, 2H), 7.51 (m, 5H), 7.15 (m, 1H), 6.83 (s, 1H), 6.48 |
| 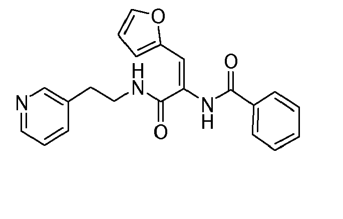 | (m, 3H), 3.60 (q, *J* = 6.7 Hz, 2H), 2.91 (q, *J* = 7 Hz, 2H); **¹³ C NMR** (100 MHz, CDCl₃): 166.5, 165.2, 150.4, 150.3, 148.0, 144.1, 136.7, 134.8, 133.3, 132.6, 129.0, 127.8, 127.7, 123.7, 114.6, 113.6, 112.3, 41.1, 33.0. LC-MS (ESI): calculated: 361.1; found: 362.2 (M+H⁺)⁺. |
| 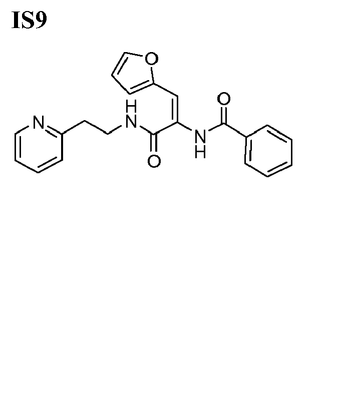 | **RMN ¹H** (400 MHz, CDCl₃): 8.041 (s, 1H), 8.18 (d, *J* = 4.3 Hz, 1H), 7.90 (d, *J* = 7.4 Hz, 2H), 7.51 (m, 5 H), 7.40 (d, *J* = 1.8 Hz,1H), 7.14 (d, *J* = 7.8 Hz,1H), 6.99 (ddd, *J*=0.9, 5.0, 7.5 Hz, 1H), 6.92 (s, 1H), 6.48 (d, *J* = 3.4 Hz, 1 H), 6.41 (dd, *J* = 1. 8, 3.5 Hz, 1H), 3.73 (dd, *J* = 5.9, 12.4 Hz, 2H), 2.99 (t, *J* = 6.3Hz, 2H); **¹³ C NMR** (100 MHz, CDCl₃): 166.3, 164.7, 160.0, 150.6, 149.1, 144.0, 136.8, 133.7, 132.4, 128.9, 127.8, 127.6, 123.8, 121.6, 114.4, 114.0, 112.3, 39.4, 36.8. LC-MS (ESI): calculated: 361.1; found: 362.2 (M+H⁺)⁺. |
| 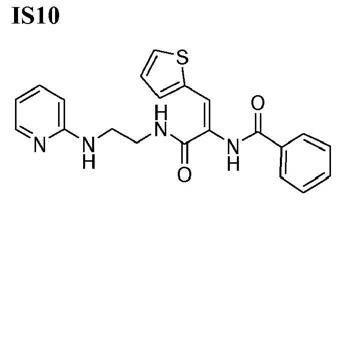 | **¹H NMR** (400 MHz, CDCl₃): 8.41 (s, 1H), 7.93 (d, *J* = 7.7Hz, 2H), 7.82 (d, *J*= 4.6Hz, 1H), 7.73 (s, 1H), 7.47 (m, 2H), 7.32 (m, 4H), 7.15 (d, *J* = 3.6 Hz, 1H), 6.99 (m, 1H), 6.44 (m, 1H), 6.32 (d, *J* = 8.4 Hz, 1H), 5.16 (s, 1 H), 3.38 (s, 4H); **¹³ C NMR** (100 MHz, CDCl₃): 167.2, 165.9, 159.0, 147.6, 137.6, 136.8, 133.2, 132.4, 132.2, 129.5, 128.8, 128.0, 127.4, 126.2, 125.1, 113.0, 108.3, 41.4, 41.3. LC-MS (ESI): calculated: 392.1; found: 393.2 (M+H⁺)⁺. |
| 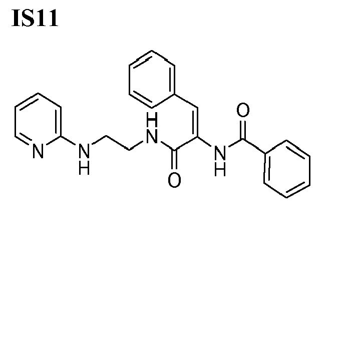 | **¹H NMR** (400 MHz, CDCl₃): 8.10 (s, 1H), 7.89 (m, 1H), 7.81 (d, *J* = 7.5 Hz,2H), 7.57 (s, 1H), 7.50 (m, 1H), 7.33 (m, 8H), 6.93 (s, 1H), 6.47 (m, 1H), 6.40 (d, *J* = 8.4Hz, 1H), 5.06 (t, *J* = 5.4 Hz, 1H), 3.53 (m, 4H); **¹³ C NMR** (100 MHz, CDCl₃): 167.0, 166.7, 158.9, 147.6, 137.4, 134.1, 133.0, 132.2, 129.9, 129.4, 128.9, 128.8, 128.7, 127.9, 127.5, 112.9, 108.4, 41.2. LC-MS (ESI): calculated: 386.2; found: 387.2 (M+H⁺)⁺. |
| **IS12** | **¹H NMR** (400 MHz, CDCl₃): 8.20 (s, 1H), 7.88 (m, 1H), 7.83 (d, *J* = 7.4 Hz, 2H), 7.56 (s, 1 H), 7.49 (m, 1H), 7.34 (m, 5 H), 6.93 (s, 1H), 6.79 (d, *J* = 8.8 Hz, 2H), 6.46 (m, 1 |
| 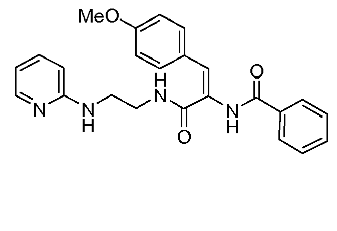 | H), 6.3 (d, *J* = 8.4 Hz, 1H), 5.11 (m, 1H), 3.48 (m, 4H); **¹³ C NMR** (100 MHz, CDCl₃): 167.3, 166.8, 160.0, 158.9, 147.6, 137.4, 133.0, 132.1, 131.2, 128.6, 128.1, 127.9, 127.7, 126.5, 114.1, 112.8, 108.4, 55.4, 41.2, 41.0. LC-MS (ESI): calculated: 416.2; found: 417.2 (M+H⁺)⁺. |
| 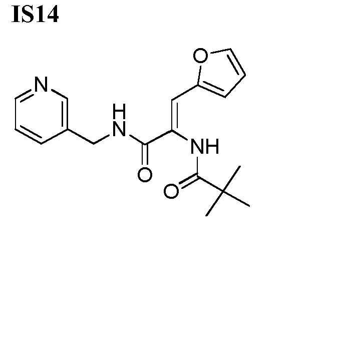 | **¹H NMR** (400 MHz, CDCl₃): 8.51 (d, *J* = 1.6 Hz, 1H), 8.46 (dd, *J* = 1.1, 4.6 Hz, 1H), 8.05 (s, 1H), 7.70 (d, *J* = 7.8 Hz, 1H), 7.45 (s, 1H), 7.23 (dd, *J* = 4.8, 7.8 Hz, 1H), 6.90 (t, *J* = 5.3Hz, 1 H), 6.73 (s, 1H), 6.46 (m, 2H), 4.52 (d, *J* = 5.9 Hz, 2H), 1.29 (s, 9H). **¹³C NMR** (100 MHz, CDCl₃): 178.2, 165.5, 150.5, 149.2, 148.9, 143.8, 135.8, 134.2, 127.9, 123.8, 114.5, 112.8, 112.4, 41.6, 39.6, 27.6. LC-MS (ESI): calculated: 327.2; found: 328.2 (M+H⁺)⁺. |
| 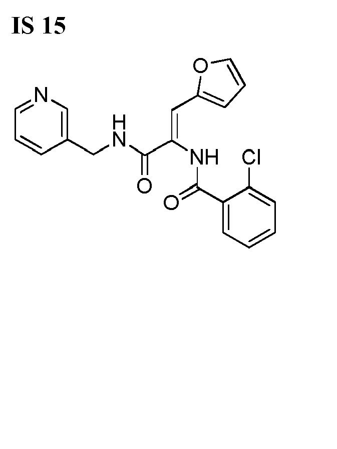 | **¹H NMR** (400 MHz, CDCl₃): 8.65 (s, 1H), 8.41 (s, 1H), 8.35 (dd, *J* = 1.5, 4.8 Hz, 1H), 7.68 (d, *J* = 7.7 Hz, 2H), 7.41 (s, 1H), 7.36 (d, *J* = 3.8 Hz, 2H), 7.31 (m, 1H), 7.17 (dd, *J* = 4.8, 7.8 Hz, 1H), 7.12 (t, *J* = 5.9 Hz, 1H), 7.06 (s, 1H), 6.56 (d, *J* = 3.4 Hz, 1H), 6.42 (dd, *J* = 1.9, 3.4 Hz, 1H), 4.49 (d, *J* = 5.8 Hz, 2H); **¹³C NMR** (100 MHz, CDCl₃): 166.0, 164.8, 150.0, 149.1, 148.7, 144.5, 135.9, 134.5, 134.0, 132.0, 131.0, 130.4, 130.1, 127.5, 125.5, 123.8, 120.2, 116.6, 115.6, 112.4, 41.6. LC-MS (ESI): calculated: 381.1; found: 382.0 (M+H⁺)⁺. |
| 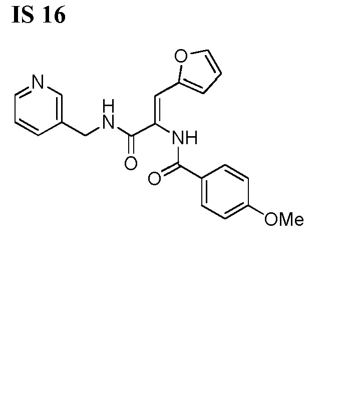 | **¹H NMR** (400 MHz, DMSO-d₆): 9.72 (s, 1H), 8.71 (t, *J* = 5.8 Hz, 1H), 8.53 (s, 1H), 8.43 (d, *J* = 4.1 Hz, 1H), 8.05 ( d, *J* = 8.5 Hz, 2H), 7.72 (m, 2H), 7.33 (dd, *J* = 4.8, 7.5 Hz, 1H), 7.19 (s, 1H), 7.06 (d, *J* = 8.7 Hz, 2H), 6.68 (d, *J* = 3.3 Hz, 1H), 6.56 (s, 1H), 4.40 (d, *J*=5.8 Hz, 2H), 3.84 (s, 3H). **¹³C NMR** (100 MHz, DMSO-d₆): 165.4, 164.7, 162.0, 149.8, 148.6, 147.8, 144.4, 135.2, 134.9, 129.9, 127.5, 126.1, 123.2, 117.6, 113.7, 113.5, 112.2, 55.4, 40.4. LC-MS (ESI): calculated: 377.1; found: 378.2 (M+H)⁺. |
| **IS17** | **¹H NMR** (400 MHz, DMSO-d₆): 9.94 (s, 1H), 8.75 (t, J = |
| 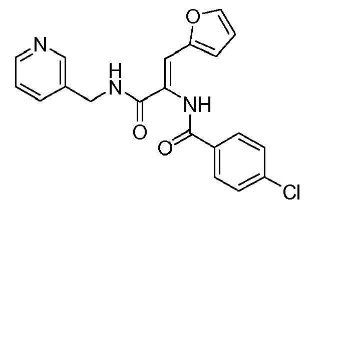 | 6.0 Hz, 1H), 8.52 (d, *J*=1.8 Hz, 1H), 8.43 (dd, *J* = 1.4, 4.6 Hz, 1H), 8.07 (d, *J* = 8.5 Hz, 2H), 7.75 (d, *J* = 1.6 Hz, 1H), 7.70 (d, *J* = 7.8 Hz, 1H), 7.62 (d, *J* = 8.5 Hz, 2H), 7.34 (dd, *J* = 4.8, 7.8 Hz, 1H), 7.23 (s, 1H), 6.72 (d, *J* = 3.4 Hz, 1 H), 6.57 (dd, *J* = 1.6, 3.3 Hz, 1H), 4.40 (d, *J* = 6.0 Hz, 2H). **¹³C NMR** (100 MHz, DMSO-d₆): 165.0, 164.4, 149.7, 148.6, 147.9, 144.8, 136.5, 135.1, 134.9, 132.7, 129.9, 128.4, 126.8, 123.3, 118.3, 114.4, 112.3, 40.3. |
| 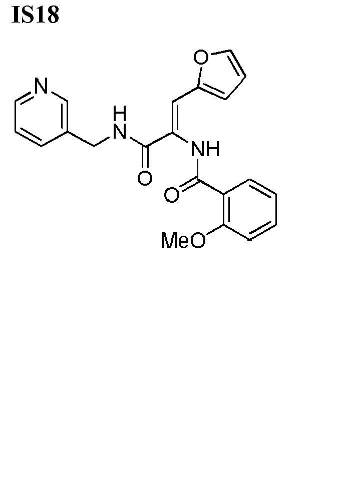 | **¹H NMR** (400 MHz, CDCl₃): 9.96 (s, 1H), 8.48 (d, *J*= 2.1 Hz, 1H), 8.39 (dd, *J* = 1.6, 4.8 Hz, 1H), 8.14 (dd, *J* = 1. 9, 7.9 Hz, 1H), 7.72 (td, *J* = 1.9, 7.8 Hz, 1H), 7.45 (m, 1H), 7.37 (d, *J* = 1.8 Hz, 1H), 7.18 (m, 2H), 7.02 (t, *J* = 7.4 Hz, 1H), 6.96 (d, *J* = 8.3 Hz, 1H), 6.80 (s, 1H), 6.45 (d, *J* = 3.4 Hz, 1H), 6.38 (dd, *J* = 1.8, 3.4 Hz, 1H), 4.50 (d, *J* = 5.9 Hz, 2H), 3.94 (s, 3H); **¹³C NMR** (100 MHz, CDCl₃): 165.7, 164.4, 157.8, 150.4, 149.2, 148.7, 143.7, 135.8, 134.3, 134.0, 132.8, 127.6, 123.6, 121.6, 120.5, 113.7, 113.5, 112.2, 111.5, 56.1, 41.4. LC-MS (ESI): calculated: 377.1; found: 378.2 (M+H)⁺. |
| 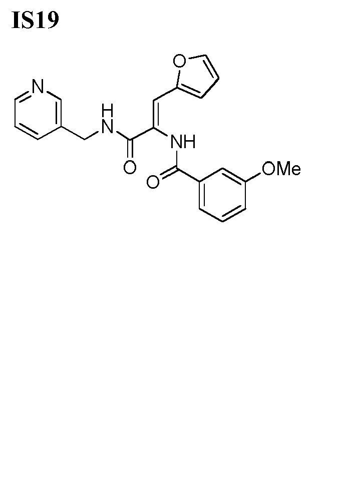 | **¹H NMR** (400 MHz, CDCl₃): 8.84 (s, 1H), 8.43 (d, *J* = 2.3 Hz, 1H), 8.34 (dd, *J* = 1.6, 4.8 Hz, 1H), 7.62 (d, *J* = 7.8 Hz, 1H), 7.48 (t, *J* = 6.0 Hz, 1H), 7.43 (m, 2H), 7.36 (d, *J* = 1.8Hz, 1H), 7.27 (t, *J* = 8.2 Hz, 1H), 7.12 (dd, *J* = 4.8, 7.8 Hz, 1 H), 7.01 (ddd, *J* = 0.8, 2.5, 8.2 Hz, 1H), 6.80 (s, 1H), 6.43 (d, *J*= 3.5 Hz, 1H), 6.36 (dd, *J* = 1.9, 3.5 Hz, 1H), 4.41 (d, *J* = 6.0 Hz, 2H), 3.76 (s, 3H); **¹³C NMR** (100 MHz, CDCl₃): 166.5, 165.6, 160.0, 150.2, 149.0, 148.6, 144.1, 135.8, 134.6, 134.3, 129.8, 127.1, 123.7, 119.7, 118.7, 114.8, 114.7, 112.9, 112.3, 55.6, 41.4. LC-MS (ESI): calculated: 377.1; found: 378.2 (M+H⁺)⁺. |

### IS4, IS5 and IS7

**IS4:** A solution of 3-aminopyridine (94 mg, 1 mmol) and azlactone **3a** (0.24 g, 1 mmol) in CH₃CN (2 mL) was heated under reflux for 3h and allowed to cool to room temperature. The precipitate, was filtered, washed with MeOH and Et₂O and recristalized in Et OH to afford 30 mg of IS4 as a white solid

| | |
|---|---|
| 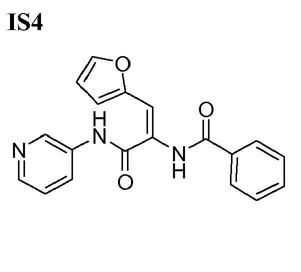 | **¹H NMR** (300 MHz, CDCl₃) : 9.28 (s, 1H), 8.72 (m, 2H), 8.23 (dd, *J* = 1.6, 4.8 Hz, 1H), 8.10 (d, *J* = 8.3 Hz, 1H), 7.93 (d, *J* = 7.2 Hz, 2H), 7.57 (m, 1H), 7.47 (t, *J* = 7.2, 1H), 7.37 (s, 1H), 7.16 (dd, *J* = 4.7, 8.3 Hz, 1H), 6.80 (s, 1H), 6.37 (m, 2H); **¹³C NMR** (100 MHz, DMSO-d₆) : 165.7, 164.0, 149.6, 144.8, 144.4, 141.9, 135.8, 133.6, 131.8, 128.4, 127.9, 127.4, 127.2, 123.4, 117.4, 114.5, 112.4. LC-MS (ESI) : calculated : 333.1 ; found 334.0 (M+H)⁺. |

**IS5:** A solution of 3-aminopyridine (47 mg, 0.5 mmol) and azlactone **3a** (120 mg, 0.5 mmol) in DMF (1 mL) was heated at 130°C for 1h and concentrated in vacuo. The residue was dissolved in AcOEt (50 ml), washed with water, a solution of Na₂CO₃ and brine. Two recristalisations in AcOEt afforded 55 mg of IS5 as a white solid.

| | |
|---|---|
| 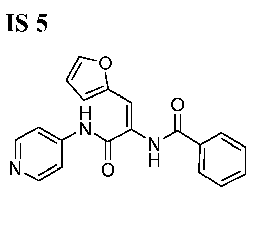 | **¹H NMR** (400 MHz, DMSO-d₆ ): 10.44 (s, 1H), 10.03 (s, 1H), 8.44 (dd, *J* = 1.6, 4.9 Hz, 2H), 8.07 (d, *J* = 7.2 Hz, 2H), 7.83 (d, *J* = 1.6 Hz, 1H), 7.72 (dd, *J* = 1.6, 4.9 Hz, 2H), 7.58 (m, 3H), 7.13 (s, 1H), 6.83 (d, *J* = 3.5 Hz, 1H), 6.63 (dd, *J* = 1.9, 3.5 Hz, 1H); **¹³C NMR** (100 MHz, CDCl₃) : 165.7, 164.5, 150.2, 149.5, 145.9, 144.9, 133.4, 131.8, 128.4, 127.9, 127.4, 117.4, 114.7, 113.9, 112.4. LC-MS (ESI): calculated: 333.1; found: 334.0 (M+H⁺)⁺. |

**IS7:** A solution of 2-hydrazinylpyridine (654 mg, 6 mmol) and azlactone **3a** (960 mg, 4 mmol) in CH₂Cl₂/EtOH (16 mL, 1/1) was heated under reflux for 1h and concentrated in vacuo. The residue was purified by flash chromatography (Et₂O followed by EtOAc) and recristalized in Et₂O to afford 0.57 g of IS7 as a white solid.

| | |
|---|---|
| 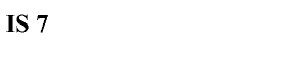 | **¹H NMR** (400 MHz, MeOD-d₄): 9.89 (s, 1H), 9.44 (s, 1H), |
| 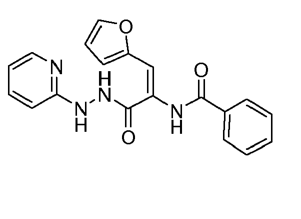 | 8.01 (m, 3H), 7.45 (m, 6H), 7.16 (s, 1H), 6.82 (d, *J* = 8.4Hz, 1H), 6.64 (m, 1H), 6.59 (d, *J* = 3.4 Hz, 1H), 6.41 (dd, *J* = 1.8, 3.5 Hz, 1H); **¹³C NMR** (100 MHz, DMSO-d₆): 165.7, 164.0, 159.2, 149.2, 146.7, 143.3, 136.9, 133.0, 131.0, 127.6, 127.2, 125.0, 116.9, 114.6, 113.6, 111.4, 106.4. LC-MS (ESI): calculated: 348.1; found: 349.0 (M+H⁺)⁺. |

### Cell culture

Coronary endothelial cells (H5V cells) were maintained in DMEM supplemented with 10% heat inactivated Fetal Calf Serum (FCS), glutamine (1mM) under 5% CO2 at 37°C.

### Induction of in vitro angiogenesis:

In order to evaluate the potential angiogenic activity of the non peptide PKR1 agonists, the test of Growth Factor Reduced Matrigel (GFR Matrigel) 24 well plate was performed. Matrigel leads to the differentiation of many cell types and induces the formation and organization of endothelial cells into capillary tubules. Twenty-four-well culture plates were coated with Matrigel and endothelial cells (H5V) were trypsin-harvested and seeded onto the coated plates at 10⁵ cells per well in the serum free assay medium with PKR1 agonists, IS1 and prokineticin-2, and incubated at 37°C for 24h. Tube formation as two dimensional branched structures were observed using an inverted phase contrast microscope (Zeiss), and the number of branching points were quantified in per well from each sample. Each experiment was repeated at least three times. Images were captured at a magnification of X10 with a digital microscope camera system (Urayama K *et al.*, 2007).

### Results

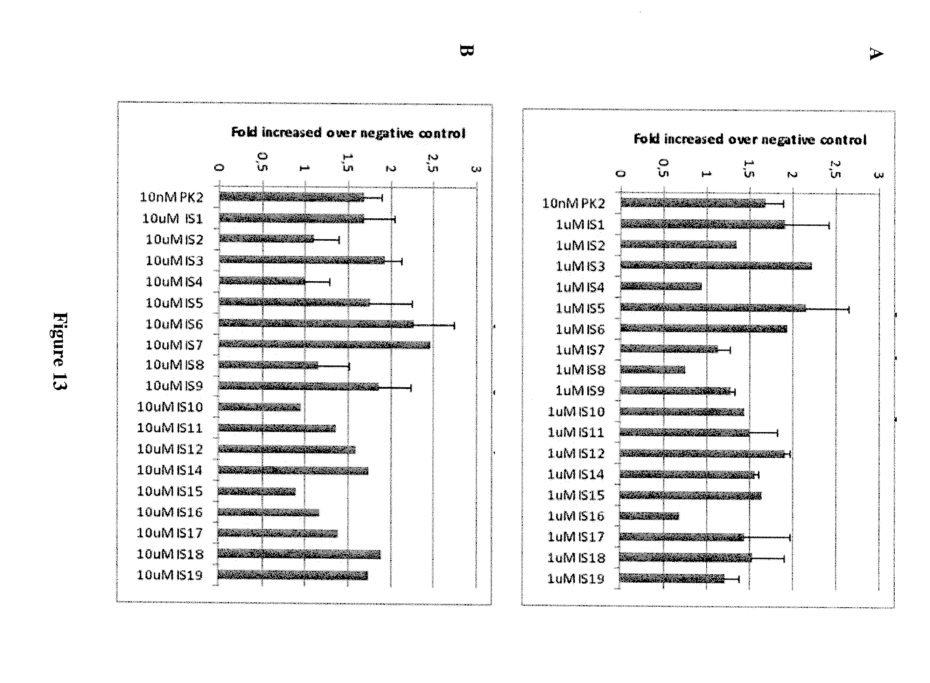

The results are presented in Figures 12 and 13. They demonstrate that the IS compounds (cf. Figure 10) are able to induce *in vitro* angiogenesis as the well known PKR1 agonist, prokineticin-2.

### Activation of PKR1 signaling pathway - Measurement of intracellular calcium

CHO-K1 (Chinese Hamster Ovary) cells were infected with an adenovirus carrying PKR1 cDNA. Cells were then stimulated, 48h after infection, with or without IS1 agonists, at 37°C.

Calcium imaging was conducted essentially as previously described (Horinouchi et al., 2007) In brief, Intracellular free calcium [Ca²⁺]i was measured with the fluorescent Ca²⁺ indicator dye Fluo 4-AM, the membrane-permeant acetoxymethyl ester form of Fluo 4 (Invitrogen). Changes in [Ca²⁺]i were determined from variations in the fluorescence intensity of Fluo 4-AM. Briefly, trypsinized CHO-K1-PKR1 cells were seeded onto glass bottom culture dishes (MatTek, MA, USA) for 24 h. Immediately before Ca²⁺ indicator loading, cells were gently washed with HBSS (invitrogen) and then incubated in Fluo 4-AM working solution (Fluo 4-AM 2.5 µM and 2.5 mM probenecid dissolved in standard buffer) for 30 min at 37 °C in cell incubator. Afterwards, cells were washed with HBSS including probenecid to remove extracellular Fluo 4-AM. Dishes were then placed on the stage of the laser confocal microscope (Leica). The fluorescence in the cells was excited at a wavelength of 488 nm and a series of images (total exposure time: 300 s) were acquired at 0.5s intervals at emission wavelength 518 nm. Data are presented as the fluorescence intensity of 3 randomly chosen cells within a randomly selected 40× field. To evaluate the effect of IS1 compound on [Ca²⁺]i in CHO-K-PKR1, the maximum change in fluorescence intensity upon addition of IS1 was measured and normalized to the baseline fluorescence obtained before IS 1 addition.

### Results

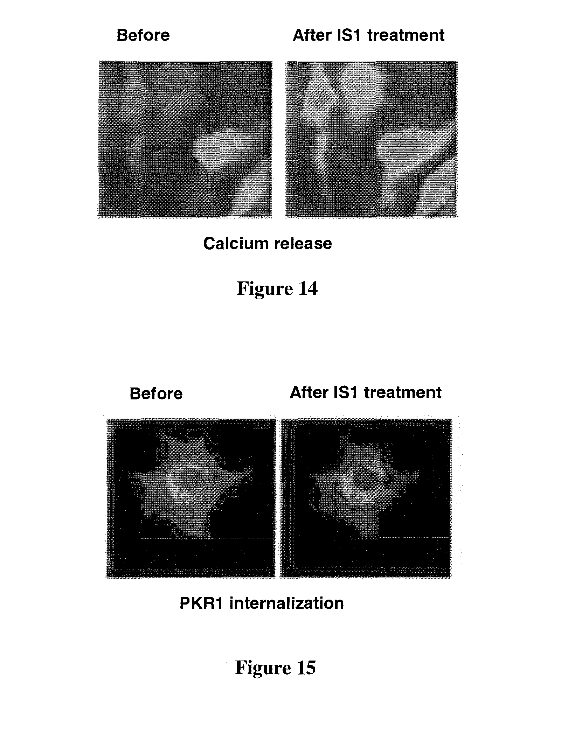

The results are presented in Figure 14. They demonstrate that the IS1 compound is able to activate the PKR1 signalling pathway in cells expressing PKR1.

### Example 3: Specificity of the agonists to PKR1 - Assessment of PKR1 internalization

The capacity of agonist compounds to induce internalization of PKR1 was assessed. Therefore, CHO-K1 cells transfected with a plasmid encoding GFP-PKR1 (Green Fluorescent Protein-PKR1), in the presence of Lipofectamine2000 (Invitrogen), were used. Cells were stimulated, 48h after transfection, with or without agonist compound, at 37°C. GFP is a fluorescent protein fusion (26.9 kDa) which has a major excitation peak at a wavelength of 396 nm and a minor one at 472 nm. Its emission peak is at 504 nm which is in the lower green portion of the visible spectrum. Fluorescence allowed tracking the localization of PKR1 after stimulation by the agonist (membrane or subcellular). Cells were seeded in glass-bottom coated with polyL-lysine and receptor studies were performed in the presence of various PKR1-ligands. Samples were observed under a Leica confocal microscope (SP2 AOBS MP) with objective 63× at 37°C. Images were automatically recorded during 20 min, with increasing time intervals to avoid bleaching effects because of repetitive scanning. Reconstituted videos contained 42 images and lasted 2 second.

### Results

The results are presented in Figure 15. They demonstrate that the IS1 compound specifically interacts with PKR1 and induces its internalization in cells expressing PKR1.

### Conclusion

These experiments demonstrate that the non peptide PKR1 agonists as described herein, and in particular IS compounds (cf. Figure 10) are able to specifically activate the PKR1 signalling pathway inducing the internalization of the receptor and angiogenesis.

### References

Cheng et al. (2002) Nature 417, 405-410
Chen et al. (2005) Mol Pharmacol 67:2070-2076
Choke et al. (2009) Eur J Vasc Endovasc Surg 37:305-310.
Dorsch et al. (2005) J. Leukoc. Biol. 78, 426-434
Erlenmeyer, F. (1893). Liebigs Ann. 275: 1-8
Hardelin & Dodé (2008) Sex Dev 2:181-193
Harlow, E. and Lane, D. (1988) Antibodies: A Laboratory Manual, ed., Cold Spring Harbor Laboratory.
Horinouchi et al. (2007) J Pharmacol Sci. 2007; 105:103-111
Kaser et al. (2003) EMBO Rep 4:469-473
Kruithof et al. Dev Biol. 2006;295:507-522
LeCouter et al. (2001) Nature 412, 877-884;
LeCouter et al. (2003) Proc. Natl. Acad. Sci.U. S. A. 100, 2685-2690
LeCouter et al. (2004) Proc. Natl. Acad. Sci. U. S. A. 101, 16813-16818
Li et al. (2001) Mol. Pharmacol. 59, 692-698;
Lin et al. (2002) J. Biol. Chem. 277, 19276-19280
Martucci et al. (2006) Br. J. Pharmacol. 147, 225-234
Masuda et al. (2002) Biochem. Biophys. Res. Commun. 293, 396-402
Negri, et al. (2005) Br. J. Pharmacol. 146, 625-632
Ng et al. (2005) Science 308, 1923-1927
Plöchl J. (1884) Chem. Ber. 17: 1623
Smart et al. (2007) Nature 445:177-182.
Soga et al. (2002) Biochim. Biophys. Acta. 1579, 173-179
Urayama et al., (2007) FASEB J. 21, 2980-2993
Wessels et al. (2004) Anat Rec A Discov Mol Cell Evol Biol. 276:43-57

## Claims

1. A prokineticin receptor-1 agonist for use for promoting the differentiation of cardial epicardin+ progenitor cells into cardiomyocytes in a subject affected with a cardiac disease and/or the differentiation of renal epicardin+ progenitor cells into vasculogenic and/or glomerular cells in a subject affected with a renal disease.

2. The prokineticin receptor-1 agonist according to claim 1, wherein the cardiac disease is selected from the group consisting of heart failure, myocardial infarction, ischemic heart disease and the cardiorenal syndrome.

3. The prokineticin receptor-1 agonist according to claim 1, wherein the renal disease is selected from the group consisting of chronic renal disease, renal artery stenosis, renal failure, acute kidney injury, acute-on-chronic renal failure, ischemic nephropathy, Churg-Strauss syndrome, Wegener's granulomatosis and the cardiorenal syndrome.

4. A prokineticin receptor-1 agonist for use for preventing fat tissue development in heart and/or kidney in a subject affected with obesity

5. An *in vitro* or *ex vivo* method of producing cardiomyocytes, wherein said method comprises the step of contacting cardial epicardin+ progenitor cells with a prokineticin receptor-1 agonist.

6. An *in vitro* or *ex vivo* method of producing glomerular cells, wherein said method comprises the step of contacting renal epicardin+ progenitor cells with a prokineticin receptor-1 agonist.

7. The prokineticin receptor -1 agonist according to any one of claims 1 to 4 and the method according to claim 5 or 6, wherein the prokineticin receptor-1 agonist is prokineticin -2 or an active fragment thereof.

8. The prokineticin receptor -1 agonist according to any one of claims 1 to 4 and the method according to claim 5 or 6, wherein the prokineticin receptor-1 agonist is a compound of formula (I) 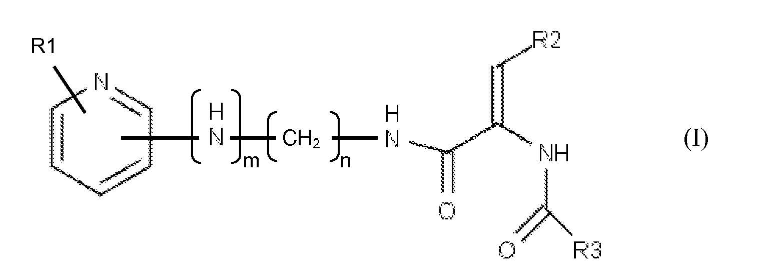 wherein
m is selected from 0 and 1;
n is selected from 0, 1 and 2;
R¹ is selected from the group consisting of a hydrogen atom; a halogen atom; a (C₁-C₆)-alkyl, a (C₂-C₆)-alkenyl and a (C₂-C₆)-alkynyl group, optionally substituted by at least one substituent selected from a hydroxyl, a carboxylic group, an aldehyde, a halogen atom and a thiol; a (C₆-C₁₂)-aryl; a 5 to 7-membered-ring heterocycle; a (C₁-C₆)-alkoxy, a (C₂-C₆)-acyl, a (C₂-C₆)-ester, a (C₁-C₆)-amine, a (C₁-C₆)-amide, a (C₁-C₆)-imine, a (C₁-C₆)-nitrile, a (C₁-C₆)-thioalkyl, a (C₁-C₆)-sulfone and a (C₁-C₆)-sulfoxide group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol;
R² is selected from the group consisting of a (C₆-C₁₂)-aryl group and a 5 to 7-membered-ring heterocycle, preferably from phenyl and furan, optionally substituted by at least one substituent selected from the group consisting of a (C₁-C₆)-alkyl, a (C₂-C₆)-alkenyl, a (C₂-C₆)-alkynyl, a (C₆-C₁₂)-aryl, a 5 to 7-membered-ring heterocycle, a (C₁-C₆)-alkoxy, a (C₂-C₆)-acyl, a (C₁-C₆)-alcohol, a carboxylic group, a (C₂-C₆)-ester, a (C₁-C₆)-amine, an amino group, a(C₁-C₆)-amide, a (C₁-C₆)-imine, a (C₁-C₆)-nitrile, a hydroxyl, an aldehyde, a (C₁-C₆)-halogenoalkyl, a thiol, a (C₁-C₆)-thioalkyl, a (C₁-C₆)-sulfone, a (C₁-C₆)-sulfoxide group and a halogen atom;
R³ is selected from the group consisting of a (C₁-C₆)-alkyl, a (C₂-C₆)-alkenyl and a (C₂-C₆)-alkynyl group, optionally substituted by at least one substituent selected from a hydroxyl, a carboxylic group, an aldehyde, a halogen atom and a thiol; and a (C₆-C₁₂)-aryl and a 5 to 7-membered-ring heterocycle, optionally substituted by at least one substituent selected from the group consisting of a (C₁-C₆)-alkyl, a (C₁-C₆)-alkoxy group and a halogen atom; or any pharmaceutically acceptable salt thereof.

9. The prokineticin receptor -1 agonist or the method according to claim 8, wherein R¹ is selected from the group consisting of a hydrogen atom; a halogen atom; a (C₁-C₃)-alkyl, optionally substituted by at least one substituent selected from a hydroxyl, a carboxylic group, an aldehyde, a halogen atom and a thiol; a (C₁-C₃)-alkoxy, a (C₂-C₃)-acyl, a (C₂-C₃)-ester, a (C₁-C₃)-amine, a (C₁-C₃)-amide and a (C₁-C₃)-thioalkyl, group wherein the alkyl part of the group is optionally interrupted by one of several heteroatoms chosen among N, O and S, and is optionally substituted by at least one substituent selected from an hydroxyl, a carboxylic group, an aldehyde, a halogen atom and a thiol.

10. The prokineticin receptor -1 agonist or the method according to claim 8, wherein R¹ is selected from the group consisting of a hydrogen atom, a halogen atom, a (C₁-C₃)-alkyl group and a (C₁-C₃)-alkoxy group.

11. The prokineticin receptor -1 agonist or the method according to any one of claims 8 to 10, wherein R² is selected from the group consisting of a phenyl group and a 5 to 7-membered-ring N-, O- or S-heterocycle, preferably a 5-membered-ring N-, O- or S-heterocycle, optionally substituted by at least one substituent selected from the group consisting of a (C₁-C₃)-alkyl, a (C₁-C₃)-alkoxy, a (C₂-C₃)-acyl, a (C₁-C₃)-alcohol, a carboxylic group, a (C₂-C₃)-ester, a (C₁-C₃)-amine, an amino group, a (C₁-C₃)-amide, a hydroxyl, an aldehyde, a (C₁-C₃)-halogenoalkyl, a thiol, a (C₁-C₃)-thioalkyl and a halogen atom.

12. The prokineticin receptor -1 agonist or the method according to any one of claims 8 to 10, wherein R² is selected from the group consisting of a phenyl, a pyrrole, a furan and a thiophene group, optionally substituted by at least one substituent selected from the group consisting of a (C₁-C₃)-alkyl and a (C₁-C₃)-alkoxy group.

13. The prokineticin receptor -1 agonist or the method according to any one of claims 8 to 12, wherein R³ is selected from the group consisting of a (C₁-C₆)-alkyl and (C₁-C₆)-amine group, optionally substituted by at least one substituent selected from a hydroxyl, a carboxylic group, an aldehyde, a halogen atom and a thiol; and a phenyl group and a 5 to 7-membered-ring N-, O- or S-heterocycle, optionally substituted by at least one substituent selected from the group consisting of a (C₁-C₃)-alkyl, a (C₁-C₃)-alkoxy group and a halogen atom.

14. The prokineticin receptor -1 agonist or the method according to any one of claims 8 to 12, wherein R³ is selected from the group consisting of a methyl, an ethyl, a propyl, an isopropyl, a butyl, an isobutyl, a tert-butyl group; and a phenyl group, optionally substituted by at least one substituent selected from the group consisting of a (C₁-C₃)-alkyl group, preferably methyl, a (C₁-C₃)-alkoxy group, preferably methoxy, and a halogen atom, preferably chlorine

15. The prokineticin receptor -1 agonist or the method according to claim 8, wherein the prokineticin receptor -1 agonist is selected from the group consisting of
| Name of agonist | Formula |
|---|---|
| IS1 | 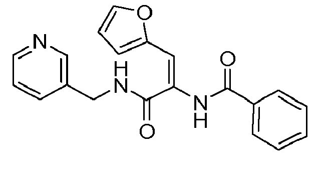 |
| IS2 | 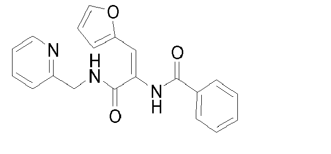 |
| IS3 | 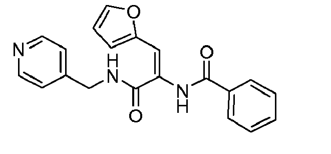 |
| IS4 | 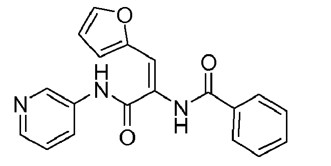 |
| IS5 | 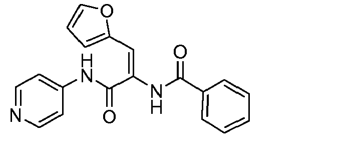 |
| IS6 | 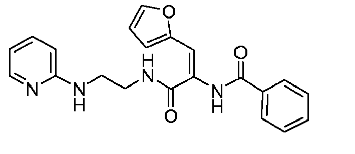 |
| IS7 | 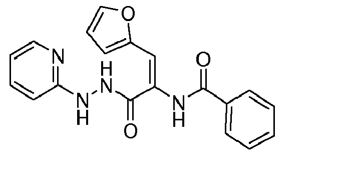 |
| IS8 | 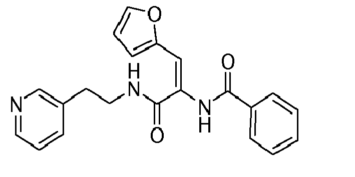 |
| IS9 | 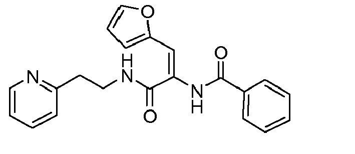 |
| IS10 | 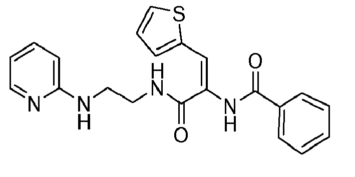 |
| IS11 | 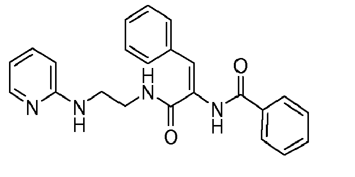 |
| IS12 | 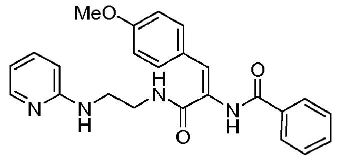 |
| IS14 | 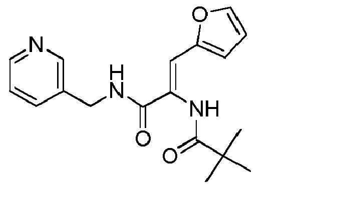 |
| IS15 | 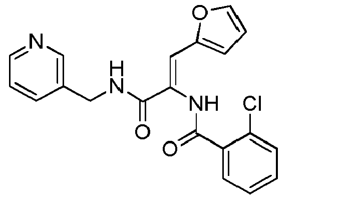 |
| IS16 | 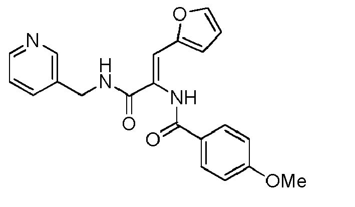 |
| IS17 | 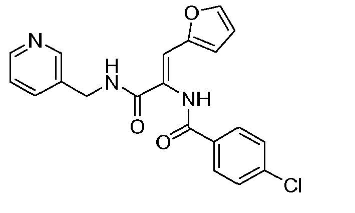 |
| IS18 | 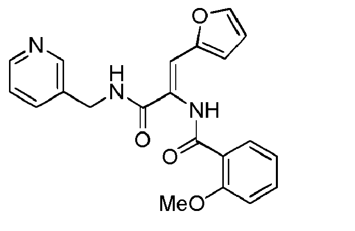 |
| IS19 | 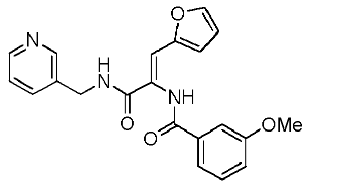 |
